# EUROPÄISCHE PATENTSCHRIFT

(11) **EP 2 995 204 B1**
(45) Veröffentlichungstag und Bekanntmachung des Hinweises auf die Patenterteilung: **28.12.2016**
(21) Anmeldenummer: 15176511.2
(22) Anmeldetag: 13.07.2015
(51) Int. Cl.: A23K 20/174, A23L 33/15, C12P 19/42

(54) **VERFAHREN ZUR HERSTELLUNG EINES NAHRUNGSMITTELS ODER EINER VORSTUFE DESSELBEN, NAHRUNGSMITTEL ODER EINE VORSTUFE DESSELBEN UND ENTSPRECHENDE VERWENDUNGEN**
METHOD FOR THE PREPARATION OF A FOOD OR A PRECURSOR OF THE SAME, FOOD OR A PRECURSOR OF THE SAME, AND USES OF SAME
PROCEDE DE FABRICATION D'UN ALIMENT OU DE SON PRECURSEUR, ALIMENT OU SON PRECURSEUR ET APPLICATIONS CORRESPONDANTES

(30) Priorität: 18.07.2014 DE 102014110182
(43) Veröffentlichungstag der Anmeldung: 16.03.2016
(73) Patentinhaber: Erdinger Weißbräu Werner Brombach GmbH & Co. KG, 85435 Erding (DE)
(72) Erfinder: Liebert, Peter, 85435 Erding (DE); Fischer, Malaika, 85435 Erding (DE); Pieczonka, Klaus, 85435 Erding (DE)
(74) Vertreter: Kuhnen & Wacker

(56) Entgegenhaltungen:
- EP-A1- 2 548 948
- WO-A1-2013/084052
- WO-A1-2014/118191

## Beschreibung

Die vorliegende Erfindung betrifft ein Verfahren zur Herstellung eines Nahrungsmittels oder einer Vorstufe desselben nach Anspruch 1, ein Nahrungsmittel oder einer Vorstufe desselben nach Anspruch 9 sowie die Verwendungen nach den Ansprüchen 10 und 11.

Vitamin B₁₂ spielt bei der Zellteilung, Blutbildung, Funktion des Nervensystems, bei mentalen Fähigkeiten sowie bei der Verstoffwechselung von Kohlenhydraten, Fetten und Eiweißen eine wesentliche Rolle. Bei einem Mangel an Vitamin B₁₂ können daher erhebliche Stoffwechselstörungen oder sonstigen körperlichen Beeinträchtigungen, wie z.B. ein verminderter Energiestoffwechsel oder ein beeinträchtigtes Immunsystem, auftreten.

Eine ausreichende Versorgung des menschlichen oder tierischen Körpers mit Vitamin B₁₂ stellt daher eine wichtige Voraussetzung für Gesundheit und Leistungsfähigkeit dar.

### Definitionen

Unter dem Begriff "Nahrungsmittel" bzw. "Lebensmittel" werden erfindungsgemäß alle Stoffe und Erzeugnisse verstanden, die der Fachmann hierunter versteht. So kann "Nahrungsmittel" alle Stoffe und Erzeugnisse umfassen, die für den menschlichen und/oder tierischen Verzehr geeignet sind und vorzugsweise eine Nährwirkung aufweisen. Insbesondere kann der Begriff "Nahrungsmittel" im Rahmen dieser Erfindung umfassen: ein getreidehaltiges Nahrungsmittel, einen getreidehaltigen Riegel, ein Malzextraktprodukt, Frühstückscerealien, eine Backware, ein Milchprodukt, ein Joghurt, ein Getränk, ein alkoholfreies Getränk, ein Bier, ein alkoholfreies Bier, ein Weizenbier, ein alkoholfreies Weizenbier, ein Biermischgetränk, ein mit einer Hefe fermentiertes Getränk, ein nicht mit Hefe fermentiertes Getränk und/oder Konzentrate der vorstehenden Nahrungsmittel. Eine Vorstufe des erfindungsgemäßen Nahrungsmittels, insbesondere des Getränks, kann insbesondere ein Sauergut, eine Maische und eine Würze sein. Im Rahmen dieser Anmeldung umfasst der Begriff "nicht fluid" in Zusammenhang mit Nahrungsmittel eine feste, pastöse, pastenartige, gelartige oder ähnliche Konsistenz eines Nahrungsmittels.

Vitamin B₁₂ ist ein Sammelbegriff für eine Reihe wasserlöslicher, strukturell ähnlicher Verbindungen mit biologischer Wirkung, die sog. Corrinoide. Aufgrund des komplex gebundenen Cobalt-Atoms werden sie als Cobalamine bezeichnet. Zu den biologisch aktiven Vitamin B₁₂-Formen zählt der Fachmann: Methylcobalamin, Desoxyadenosylcobalamin, Hydroxycobalamin und Sulfitcobalamin. Von diesen werden Methylcobalamin und Desoxyadenosylcobalamin als die biologisch wirksamsten bzw. aktivsten Formen angesehen. Von dieser Gruppe ist Cyanocobalamin nur auf künstlichem, d.h. synthetischem Wege zugänglich.

Neben diesen aktiven Vitamin B₁₂-Formen gibt es auch sogenannte inaktive Analoga. Diese werden auch Pseudo-Vitamin B₁₂ genannt, weil sie zwar eine ähnliche chemische Struktur wie das echte Vitamin B₁₂ aufweisen, aber unter Umständen keine Vitamin-Wirkung für den menschlichen oder tierischen Körper entfalten. Sie sind nicht geeignet, physiologische Aufgaben des Vitamins B₁₂ im Organismus zu erfüllen. Weiterhin können sie die Aufnahme und Verstoffwechselung von biologisch aktivem Vitamin B₁₂ blockieren.

Unter Pseudo-Vitamin B₁₂ wird im Rahmen dieser Anmeldung insbesondere 7-Adeninyl-Cyanocobamide verstanden.

Im Rahmen dieser Anmeldung wird die Definition des Begriffs "Vitamin B₁₂" auf die im menschlichen oder tierischen Organismus aktiven Formen des Vitamins B₁₂ oder deren Vorstufen beschränkt. Darüber hinaus ist der Begriff "Vitamin B₁₂" im Rahmen dieser Anmeldung auf Formen des Vitamin B₁₂ beschränkt, welche sich mikrobiologisch erzeugen lassen. Da sich das vorstehend erwähnte Cyanocobalamin nur auf synthetischem Wege erzeugen lässt und daher kein "natürliches" Vitamin B₁₂ darstellt, ist diese Form des Vitamin B₁₂ von dem Begriff "Vitamin B₁₂" im Rahmen dieser Anmeldung nicht umfasst.

Damit beschränkt sich die Definition des Begriffs "Vitamin B₁₂" im Rahmen dieser Anmeldung auf die nachfolgenden Vitamin B₁₂-Spezies: Methylcobalamin, Desoxyadenosylcobalamin, Hydroxycobalamin und Sulfitcobalamin.

Die Vertreter der vorstehend genannten Gruppe zeichnen sich im Allgemeinen durch eine hohe Bioverfügbarkeit aus, so dass diese geeignet sind, die Versorgung des menschlichen oder tierischen Körpers mit Vitamin B₁₂ zu verbessern.

Im Rahmen dieser Anmeldung wird der Begriff "Bioverfügbarkeit" als die Resorption des Nährstoffs (Vitamin B₁₂) vom Gastrointestinaltrakt ins Blut verstanden, wodurch dieser in den systemischen Kreislauf gelangt und somit den Zellen/Organen zur Verfügung steht. Des Weiteren wird auf die Definition des Begriffs "bioverfügbar" oder "Bioverfügbarkeit" auf Absatz [0013] der Beschreibung der Internationalen Patent-Offenlegungsschrift WO 2012/109 324 A1 verwiesen. Auf letztgenanntes Dokument wird hiermit vollinhaltlich Bezug genommen.

Zur Bestimmung von bioverfügbarem Vitamin B₁₂ wurde eine Nachweisanalytik auf Basis des sog. ADVIA-Systems eingesetzt. Dieser Bioverfügbarkeitsnachweis basiert auf dem sogenannten Intrinsic Factor (IF) und wird bspw. zum Nachweis von bioverfügbarem Vitamin B₁₂ im Blut verwendet. Vitamin B₁₂ wird im letzten Dünndarmabschnitt (Ileum) mittels des Intrinsic Factors (IF = Protein, welches in den Parietalzellen der Magenschleimhaut sezerniert wird) absorbiert. Im Rahmen dieses Tests wird das IF-ungebundene Vitamin B₁₂ als nicht bioverfügbar definiert, da dieses nicht in den Blutkreislauf und hiermit einhergehend nicht an das entsprechende Gewebe gelangt.

In der vorliegenden Anmeldung wurde der ADVIA Centaur VB12-Test (111659 Rev. N, 2008-09; VB12 2/12) zum Nachweis von bioverfügbarem Vitamin B₁₂ verwendet. Dieser ist ein kompetitiver Immunoassay unter Anwendung der direkten Chemilumineszenz-Technologie. Dabei konkurriert Vitamin B₁₂ in der Probe mit dem mit Acridiniumester markierten Vitamin B₁₂ in einem Lite-Reagenz um eine begrenzte Menge an gereinigtem IF, der kovalent an paramagnetische Partikel in der Solid Phase gebunden ist. In diesem Test werden das Releasing Agens (Natriumhydroxid) und DTT zur Freisetzung von Vitamin B₁₂ aus endogenen Bindungsproteinen in der Probe verwendet. Zugesetztes Cobinamid verhindert eine Rückbindung nach der Zugabe der Solid Phase zur Probe.

Zur Bestimmung der (Gesamt-)Konzentration an Vitamin B₁₂, welche alle Formen des Vitamins B₁₂, also bioverfügbare und nicht-bioverfügbare Formen, erfasst, wird im Rahmen dieser Anmeldung die Messmethode r-Biopharm AOAC-Methode Nr. 101002 verwendet. Alternativ kann zur Bestimmung der Gesamt-Konzentration die Methode "Fresenius AOAC-Methode Nr. 952.20" verwendet werden.

In Zusammenhang mit einem Hefeerzeugnis wird erfindungsgemäß unter dem Begriff "Autolysat" ein meist flüssiges Nährsubstrat verstanden, das durch Auflösen der Zellen von Backhefe, Futterhefe (Eiweißhefe) und/oder insbesondere von Bierhefe durch zelleigene Enzyme gewonnen wird.

In Zusammenhang mit einem Hefeerzeugnis wird erfindungsgemäß unter dem Begriff "Extrakt" ein meist pulver-, gel- oder pastenartiges Erzeugnis aus Hefeautolysat (mittels eigenen Hefeenzymen erzeugt) oder Hefehydrolysat (mittels Fremdenzymen erzeugt) mit einem hohen Gehalt pro Trockenmasse an Aminosäuren (bspw. 30 bis 50 %), Kohlenhydraten (bspw. 20 bis 30 %) und Vitaminen (insbesondere: B-Gruppe: Thiamin, Riboflavin, Nicotinsäure) verstanden. Allgemein wird ein Hefeextrakt hergestellt, indem ein Hefeautolysat oder -hydrolysat von den unlöslichen Zellbestandteilen wenigstens teilweise befreit, aufkonzentriert und gegebenenfalls sprühgetrocknet wird. Typischerweise weist ein Hefeextrakt einen Trockensubstanzgehalt von 70 bis 80 % bei pastenartiger Konsistenz und von 95 bis 97 % bei Pulverkonsistenz auf.

In Zusammenhang mit einem Hefeerzeugnis wird erfindungsgemäß unter dem Begriff "getrocknete Form" jede Gestalt einer Hefe verstanden, welche einen Wassergehalt von höchstens 5 %, vorzugsweise höchstens 2 %, insbesondere höchstens 1 %, aufweist. Insbesondere wird hierunter eine getrocknete Hefe in Gestalt von Flocken oder Pulver oder eine gefriergetrocknete Hefe verstanden.

Dem Begriff "Sauergut" wird im Rahmen dieser Erfindung die übliche, im Gebiet der Brauereitechnologie geläufige Definition zugeordnet. Hierunter wird insbesondere ein zur pH-Absenkung mikrobiell, vorzugsweise mit Milchsäurebakterien, behandeltes Nährmedium, insbesondere eine Maische und/oder Würze, verstanden.

Im Rahmen dieser Anmeldung wird unter den Begriffen "Maische", "Würze" und "Glattwasser" insbesondere die Substrate verstanden, welche der Fachmann unter derselben Bezeichnung aus dem Bierbereitungsprozeß kennt. Die Begriffe sind aber erfindungsgemäß nicht notwendigerweise hierauf beschränkt, sondern können sich auch auf analoge Substrate, also Vorstufen, hinsichtlich sonstiger Getränke oder anderer Nahrungsmittel, wie beispielsweise Whiskey-Maischen, beziehen. Insbesondere kann erfindungsgemäß die Würze eine aus einer erfindungsgemäßen Maische, wie hierin definiert, hergestellt werden.

Vorzugsweise kann der Begriff "Maische" erfindungsgemäß auf eine Maische beschränkt sein, welche unter Verwendung eines kohlenhydrathaltigen Substrats oder eines Gemisches von kohlenhydrathaltigen Substraten hergestellt wurde. Dabei weist das kohlenhydrathaltige Substrat oder das Gemisch von kohlenhydrathaltigen Substraten einen Anteil an Braumalz von wenigstens 80 Massen-%, vorzugsweise wenigstens 90 Massen-%, vorzugsweise wenigstens 95 Massen-%, vorzugsweise wenigstens 98 Massen-%, vorzugsweise wenigstens 99 Massen-%, insbesondere etwa 100 Massen-%, auf.

Insbesondere kann der Begriff "Maische" erfindungsgemäß auf eine Maische beschränkt sein, welche unter Verwendung von Braumalz hergestellt wurde, wobei das Braumalz einen Anteil an Weizenmalz von wenigstens 50 Massen-%, vorzugsweise wenigstens 52 Massen-%, insbesondere wenigstens 55 Massen-%, aufweist. Erfindungsgemäß schließt der Begriff "Braumalz" Malze einer Sorte und auch Mischungen verschiedener Malzsorten ein.

Ferner kann der Begriff "Würze" erfindungsgemäß auf eine Würze beschränkt sein, welche aus einer Maische gewonnen wurde, welche unter Verwendung von Braumalz mit einem Anteil an Weizenmalz von wenigstens 50 Massen-%, vorzugsweise wenigstens 52 Massen-%, insbesondere wenigstens 55 Massen-%, hergestellt wurde.

Unter dem Begriff "Hopfenbitterstoffe" werden im Rahmen dieser Anmeldung alle dem Fachmann bekannten Bitterstoffe des Hopfens und Hopfenharze verstanden. Hierzu gehören sowohl die Weichharze als auch die Hartharze, einschließlich der Bittersäuren, insbesondere der α-Säuren und β-Säuren, und die bekannten Derivate dieser Harze und Säuren, insbesondere deren Oxidationsprodukte.

Unter dem Begriff "frei von Hopfenbitterstoffen" in Bezug auf ein Medium (z.B. Nährmedium oder Hefeerzeugnis) wird im Rahmen dieser Anmeldung die vollständige Abwesenheit von Hopfenbitterstoffen in diesem Medium verstanden.

Unter dem Begriff "im Wesentlichen frei von Hopfenbitterstoffen" in Bezug auf ein erstes Nährmedium wird im Rahmen dieser Anmeldung ein Gehalt an Hopfenbitterstoffen von höchstens 15 %, vorzugsweise höchstens 10 %, vorzugsweise höchstens 5 %, insbesondere höchstens 2 %, bezogen auf den Gehalt an Hopfenbitterstoffen, den eine brauereiübliche Anstellwürze für eine Vergärung mit einer untergärigen oder obergärigen Hefe mit Bittereinheiten (EBC-Methode) im Bereich von 15 bis 38, vorzugsweise 20 bis 35, aufweist. Dabei können erfindungsgemäß die vorstehenden Prozentangaben auch auf jede Einzelsubstanz der Gruppe der Hopfenbitterstoffe (z.B. Humulon oder Lupulon) angewandt werden.

Unter dem Begriff "im Wesentlichen frei von Hopfenbitterstoffen" in Bezug auf ein Hefeerzeugnis wird im Rahmen dieser Anmeldung ein Gehalt an Hopfenbitterstof-fen von höchstens 20 %, vorzugsweise höchstens 15 %, vorzugsweise höchstens 10 %, vorzugsweise höchstens 5 %, insbesondere höchstens 2 %, bezogen auf den Gehalt an Hopfenbitterstoffen, den eine untergärige oder obergärige Betriebshefe in einer Brauerei, insbesondere eine frische Erntehefe der ersten, zweiten oder dritten Führung, aufweist, die während einer Vergärung einer brauereiübliche Würze mit Bittereinheiten (EBC-Methode) im Bereich von 15 bis 38, vorzugsweise 20 bis 35, geerntet wurde. Dabei können erfindungsgemäß die vorstehenden Prozentangaben auch auf jede Einzelsubstanz der Gruppe der Hopfenbitterstoffe (z.B. Humulon oder Lupulon) angewandt werden.

In analoger Weise zu den vorstehenden Definitionen ist im Rahmen dieser Anmeldung auch der Begriff "Hopfenbitterstoffe ... vollständig oder im Wesentlichen vollständig entfernt ..." bezogen auf eine Hefe oder ein Hefeerzeugnis zu verstehen.

Der Gegenstand der Erfindung kann sich auf die Herstellung von alkoholischen und nicht alkoholischen Getränken in der Brauerei, insbesondere von Bier, und die entsprechenden Erzeugnisse beziehen. Im Rahmen dieser Anmeldung wird dann den Begriffen "Vorderwürze", "Kochen", "Heißhalten", "Trub", "Anstelltemperatur", "Rein-zuchthefe", "Erntehefe", "Waschen der Hefe", "Verarbeiten zu einem Getränk", "Würzesäuerung", "Maischesäuerung" usw. jeweils die Bedeutung zugeordnet, die ein Fachmann im Bereich der Getränkeherstellung, insbesondere im Bereich der Bierherstellung, dem jeweiligen Fachbegriff oder der Tätigkeit üblicherweise zuordnet. Analoges gilt bei der Verwendung von Begriffen für Gegenstände oder Tätigkeiten innerhalb dieser Anmeldung, die sich auf die Herstellung oder Behandlung von festen oder pastösen bzw. gelartigen Nahrungsmitteln und die entsprechenden Erzeugnissen beziehen.

Unter "Behandlung" oder "Behandeln" mit Milchsäurebakterien oder Hefe wird im Rahmen dieser Anmeldung jede Art der teilweisen oder vollständigen Verstoffwechselung von aus einem Nährmedium stammenden Nährstoffen, insbesondere eine teilweise oder vollständige Vergärung, durch Milchsäurebakterien, verstanden. Darüber hinaus kann "Behandeln" jede Art des Inkontaktbringens oder Inkontaktseins von Mikroorganismen, vorzugsweise Milchsäurebakterien oder Hefe, insbesondere von den in dieser Anmeldung erfindungsgemäß vorgesehenen Milchsäurebakterien und Bierhefen, mit einem Nährmedium, sein.

Der Begriff "Milchsäurebakterien" im Sinne dieser Anmeldung umfasst jede Art und Zustand von Milchsäurebakterien, d.h. jede beliebige Art bzw. Unterart bzw. jeden beliebigen Stamm, soweit dies in dieser Anmeldung nicht weiter beschränkt ist. Ferner umfasst dieser Begriff lebende und/oder tote Bakterienzellen, insbesondere lebende Bakterienzellen. Die in dieser Anmeldung angegebenen DSMZ-Nummern dienen lediglich einer eindeutigen Identifizierung der erfindungsgemäß verwendeten Bakterienarten oder -unterarten. Die Erfindung ist jedoch nicht auf die DSMZ als alleinige Bezugsquelle für diese erfindungsgemäß verwendeten Bakterienarten oder -unterarten beschränkt.

Erfindungsgemäß wird unter "Weizenbier" ein obergäriges Bier mit einem Weizenmalzanteil von wenigstens 50 Massen-%, vorzugsweise wenigstens 52 Massen-%, insbesondere wenigstens 55 Massen-%, in der Schüttung oder im kohlenhydrathaltigen Substrat verstanden.

Unter dem Begriff "alkoholfrei" wird in Bezug auf ein Nahrungsmittel, Getränk oder Bier oder ein sonstiges in dieser Anmeldung genanntes Erzeugnis erfindungsgemäß ein Gehalt an Ethanol im Erzeugnis von 0 bis weniger als 1,2 Volumen-%, vorzugsweise von 0 bis weniger als 1,0 Volumen-%, vorzugsweise von 0 bis weniger als 0,5 Volumen-%, vorzugsweise von 0 bis weniger als 0,3 Volumen-%, vorzugsweise von 0 bis weniger als 0,1 Volumen-%, insbesondere etwa 0 Volumen-%, verstanden.

Im Rahmen dieser Anmeldung wird unter den Angaben "etwa", "ungefähr" oder ähnlichem eine relative Abweichung vom jeweiligen Bezugswert von höchstens 10 %, vorzugsweise höchstens 5 %, vorzugsweise höchstens 3 %, insbesondere höchstens 1 %, verstanden.

Die in dieser Anmeldung verwendeten Volumen- oder Volumenanteils-Angaben beziehen sich immer auf die Temperatur oder Temperaturen, welche ein Fachmann dem darauf bezogenen Fluid oder Gemisch für den jeweiligen Verwendungszweck bzw. beim jeweiligen Verfahrensschritt typischerweise zuordnen würde, soweit eine Temperatur nicht explizit angegeben ist.

Alle in der Anmeldung angegebenen Massen und Massenkonzentrationen beziehen sich jeweils auf die Trockenmasse des betreffenden Stoffs.

### Stand der Technik

Das Dokument EP 0 545 139 B1 offenbart ein Malzgetränk, welches einen unvergorenen Malzsud mit einem Stammwürzegehalt zwischen 2 und 15 % aufweist. Dieses Malzgetränk kann leicht gehopft sein und weist einen Gehalt an Cyanocobalamin im Bereich von 5 bis 30 Mikrogramm pro Liter auf.

Im Dokument EP 824 152 B1 wird die Erzeugung von natürlichem Vitamin B₁₂ in relativ hohen Konzentrationen (≥ 0,1 Gew.-%) unter Verwendung von *Propionibacterium freudenreichii* beschrieben.

In ähnlicher Weise betrifft GB 793 467 A die Erzeugung von Zubereitungen, welche eine hohe Vitamin B₁₂-Aktivität insbesondere durch Kultivierung eines bestimmten Typs von *Propionibacterium,* insbesondere *P. freudenreichii* oder *P. shermanii,* aufweisen. Hierbei wird ein Verfahren zur Erzeugung einer physiologisch aktiven Zubereitung von Vitamin B₁₂ offenbart, welche frei von Pseudo- oder inaktiven Vitamin B₁₂-Varianten ist.

Ähnliche Lehren sind ferner den Dokumenten GB 925 526 A und GB 1 007 972 A zu entnehmen.

Das Dokument US 6 492 141 B1 befasst sich mit der industriellen Herstellung von Vitamin B₁₂ unter Verwendung von *Propionibacterium.*

Das Dokument US 2006/0 051323 A1 offenbart ein Lebensmittel, welches Partikel aufweist, die eine mikrobielle Biomasse und einen festen Träger aufweist, wobei die Biomasse Vitamin B₁₂ enthält. Dabei kann die Biomasse beispielsweise *Propionibacterium,* vorzugsweise *P. freudenreichii,* umfassen. In sprühgetrockneter Form kann die derart erzeugte Biomasse als Lebensmittelzusatz verwendet werden.

Das Dokument WO 2012/143 469 A1 offenbart ein Lebensmittelprodukt in Rie-gelform, das feste Partikel, beispielsweise bestehend aus Getreidekörnern, sowie Vitamin B₁₂ in Form verschiedener Cobalamine enthält.

Das Dokument WO 2009/124 529 A2 offenbart ein Verfahren zur Herstellung eines Erfrischungsgetränks, welches mit mikrobiologisch gebildetem Cobalamin durch das zusätzliche Animpfen mit einem Cobalamin synthetisierenden Mikroorganismusstamm erreicht wird. Dabei sind als Cobalamin synthetisierende Mikroorganismusarten wie beispielsweise *Torulopsis glabrata, Lactobacillus lactis (lactis) und Kluyveromyces lactis* besonders bevorzugt.

Dokument JP 5814629 2 A lehrt die mikrobielle Erzeugung von Vitamin B₁₂ in hoher Ausbeute unter Verwendung von *Propionibacterium freudenreichü* und *Propionibacterium shermanii.*

Die unveröffentlichte Patentanmeldung DE 10 2013 100891.7 offenbart ein Verfahren zur Herstellung eines Sauergutes, einer Maische, einer Würze, eines Getränks, eines Nahrungsmittels, eines Weichwassers und eines Malzes und die entsprechenden Erzeugnisse. Dabei enthalten die Erzeugnisse Vitamin B₁₂, welches wenigstens zu einem hohen Anteil für den menschlichen und/oder tierischen Körper bioverfügbar ist.

Die Offenlegungsschrift EP 2 548 948 A1 offenbart ein Produkt, erhältlich durch Milchsäuregärung eines Substrats durch einen Stamm der Spezies *Lactobacillus plantarum,* insbesondere FERM ABP-11349 oder FERM ABP-11350, der eine Zellzahl von 10⁸ KBE/g auf reinem Orangen- oder Grapefruitsaft erreichen kann.

Die Offenlegungsschrift WO 2013/084 052 A1 offenbart ein Produkt, erhältlich durch Milchsäuregärung eines Substrats durch einen Stamm der Spezies *Lactobacillus reuteri* als Vitamin B₁₂-Erzeuger, ausgewählt aus der Gruppe, bestehend aus DSM 23877, DSM 23878, DSM 23879 und DSM 23880.

Die Offenlegungsschrift WO 2014/118 191 A1 offenbart ein Verfahren zur Herstellung eines Nahrungsmittels mit den Schritten des Bereitstellens einer Maische oder einer Würze oder eines Glattwassers als ein erstes Nährmedium; und des Behandelns des ersten Nährmediums mit Milchsäurebakterien der Art *Lactobacillus coryniformis,* der Art *Lactobacillus backii* (DSMZ Nr. 18080), der Art *Lactobacillus plantarum* oder der Art *Lactobacillus fermentum* (DSMZ Nr. 20052) oder mit einem Gemisch aus Milchsäurebakterien von wenigstens zwei dieser Arten.

Allerdings wäre es wünschenswert, wenn die Menge oder Konzentration an Vitamin B₁₂, welche in den Erzeugnissen, die mit dem zuletzt diskutierten Verfahren erzeugt werden, enthalten ist, weiter gesteigert werden könnte.

Auf die unveröffentlichte Patentanmeldung DE 10 2013 100891.7 wird hiermit vollinhaltlich Bezug genommen, so dass deren gesamte Offenbarung, insbesondere die darin genannten technischen Merkmale und Wirkungen, Teil der Offenbarung dieser Anmeldung und der hierin beschriebenen Erfindung wird.

### Aufgabe der Erfindung

Aufgabe der vorliegenden Erfindung ist es, ein verbessertes Verfahren zur Herstellung eines Nahrungsmittels oder einer Vorstufe desselben, insbesondere eines Sauerguts, einer Maische, einer Würze, eines Getränks oder eines nicht fluiden Nahrungsmittels, und das entsprechende Nahrungsmittel oder eine Vorstufe desselben bereitzustellen, wobei dieses Nahrungsmittel oder bereits eine Vorstufe davon geeignet ist, die Vitamin B₁₂-Versorgung des menschlichen und/oder tierischen Körpers bei Aufnahme dieses Nahrungsmittel oder einer Vorstufe desselben zu verbessern.

Es ist ein Aspekt der vorliegenden Erfindung, ein Nahrungsmittel oder eine Vorstufe desselben bereitzustellen, welches oder welche eine gesteigerte Menge oder Konzentration an Vitamin B₁₂ enthält.

Es ist ein Aspekt der vorliegenden Erfindung, ein Nahrungsmittel oder eine Vorstufe desselben bereitzustellen, welches oder welche auf natürliche Weise hergestellt worden ist und/oder dessen Rohstoffe nach dem deutschen Reinheitsgebot zulässig sind bzw. dem deutschen Reinheitsgebot entsprechen.

Es ist ein weiterer Aspekt dieser Erfindung, die vorstehend definierte Aufgabe und/oder wenigstens einen der vorstehend definierten Aspekte auf einfache technische Weise und kostengünstig, insbesondere mit den in einer Brauerei herkömmlich vorhandenen Ausstattung, zu verwirklichen.

### Zusammenfassung der Erfindung

Die erfindungsgemäße Aufgabe wird durch die Gegenstände der Ansprüche 1 bis 13 wie auch durch die nachfolgend definierten Gegenstände gelöst.

In verfahrenstechnischer Hinsicht wird die erfindungsgemäße Aufgabe durch ein Verfahren zur Herstellung eines Nahrungsmittels oder einer Vorstufe desselben gemäß Anspruch 1 gelöst. Dabei weist das Verfahren wenigstens die nachfolgenden Schritte auf:
(a) Bereitstellen eines ersten Nährmediums, vorzugsweise einer Maische oder einer Würze, insbesondere einer Vorderwürze, oder eines Glattwassers; und
(b) Behandeln des ersten Nährmediums mit Milchsäurebakterien der Art *Lactobacillus coryniformis,* insbesondere der Unterart *Lactobacillus coryniformis* subsp. *coryniformis* (DSMZ Nr. 20007), der Art *Lactobacillus backii* (DSMZ Nr. 18080), der Art *Lactobacillus plantarum* (vorzugsweise DSMZ Nr. 2601 oder 2648 oder 13273), insbesondere der Unterarten *Lactobacillus plantarum* subsp. *plantarum* (DSMZ Nr. 20174) oder *Lactobacillus plantarum* subsp. *argentoratensis* (DSMZ Nr. 16365), oder der Art *Lactobacillus fermentum* (DSMZ Nr. 20052) oder mit einem Gemisch aus Milchsäurebakterien von wenigstens zwei dieser Arten oder Unterarten.

Dabei findet die Behandlung gemäß Schritt (b) in Anwesenheit eines Hefeerzeugnisses statt, wobei das Hefeerzeugnis ein Extrakt, ein Autolysat und/oder eine getrocknete Form einer Hefe enthält oder aus einem Extrakt, einem Autolysat und/oder einer getrockneten Form einer Hefe besteht. Erfindungsgemäß kann das Hefeerzeugnis auch ein beliebiges Gemisch aus einem Extrakt, einem Autolysat und/oder einer getrockneten Form einer Hefe sein oder enthalten. Das Hefeerzeugnis liegt vorzugweise in einer Massenkonzentration im Bereich von ≥ 0,2 und ≤ 20 g/l bezogen auf das erste Nährmedium vor.

Durch die Behandlung des ersten Nährmediums mit den vorstehend genannten Milchsäurebakterien wird ein Medium, insbesondere ein Sauergut, erhalten, das Vitamin B₁₂ enthält.

Überraschenderweise kann durch die Anwesenheit eines Hefeerzeugnisses oder einer Kombination der beschriebenen Hefeerzeugnisse während der Behandlung des ersten Nährmediums mit Milchsäurebakterien der genannten Arten die Menge und/oder Massenkonzentration an erzeugtem Vitamin B₁₂ im ersten Nährmedium gesteigert werden.

Insbesondere ab einer Zugabe oder Massenkonzentration des Hefeerzeugnisses von etwa 0,2 g/l (bezogen auf das erste Nährmedium) kann eine Steigerung der erzeugten Menge und/oder Massenkonzentration an Vitamin B₁₂ beobachtet werden, auch wenn das Hefeerzeugnis als solches kein Vitamin B₁₂ enthält. Dabei nimmt die zusätzlich erzeugte Menge an Vitamin B₁₂ mit der Massenkonzentration des bei der Behandlung anwesenden Hefeerzeugnisses zumindest über einen bestimmten Konzentrationsbereich in etwa linear zu.

Jenseits einer Massenkonzentration des Hefeerzeugnisses von etwa 20 g/l (bezogen auf das erste Nährmedium) tritt eine starke Sedimentbildung im Reaktionsgefäß auf, und es kann zu Qualitätseinbußen bei dem erzeugten Nahrungsmittel (oder einer Vorstufe desselben) hinsichtlich des Geruchs und Geschmacks kommen. Dies ist vermutlich auf eine Überfrachtung mit Zellmaterial und deren Abbauprodukten zurückzuführen.

Wenn das Hefeerzeugnis eine getrocknete Hefe ist, kann es bei hohen Zugaben, insbesondere bei Zugaben jenseits der 20 g/l zu unangenehmer Geruchsentwicklung kommen. Dies ist vermutlich auf die verstärkte Freisetzung von Fettsäuren und/oder deren Abbauprodukten zurückzuführen. Zudem wird die technische Umsetzung des beanspruchten Verfahrens beim Einsatz großer Mengen an Hefeerzeugnissen, insbesondere bei Massenkonzentrationen jenseits der 20 g/l, zunehmend unwirtschaftlich.

Erfindungsgemäß kann das erste Nährmedium beispielsweise eine Nährbouillon, eine Maische, eine Würze, vorzugsweise eine Vorderwürze, oder ein Glattwasser sein.

Insbesondere bei der Verwendung von Maische oder Würze als erstem Nährmedium hat die Anmelderin überraschend festgestellt, daß die erfindungsgemäß eingesetzten Milchsäurebakterien in einem derartigen Milieu und gerade in Anwesenheit eines Hefeerzeugnisses vermehrt Vitamin B₁₂ erzeugen und damit zu einer Erzeugung von Vitamin B₁₂ geeignet sind. Ferner wurde festgestellt, daß das mittels der erfindungsgemäß eingesetzten Milchsäurebakterien erzeugte Vitamin B₁₂ auch bioverfügbar in Sinne dieser Anmeldung ist.

Bei Anwendung des erfindungsgemäßen Verfahrens kann also eine mengenmäßig nochmals gesteigerte Erzeugung von Vitamin B₁₂, insbesondere bioverfügbarem Vitamin B₁₂, erreicht werden, offensichtlich ohne daß ein "Umschalten" des Stoffwechsels der erfindungsgemäß vorgesehenen Milchsäurebakterien auf die Erzeugung von nicht gewünschten Substanzen stattfindet. Ein derartiges Umschalten ist beispielsweise von Milchsäurebakterien anderer Arten, wie beispielweise *Lactobacillus reuterii,* bekannt.

Vorteilhafte Ausführungsformen des erfindungsgemäßen Verfahrens sind Gegenstand der abhängigen Ansprüche.

So kann das erste Nährmedium frei oder im Wesentlichen frei von Hopfenbitterstoffen sein; und/oder das Hefeerzeugnis frei oder im Wesentlichen frei von Hopfenbitterstoffen sein.

Das erste Nährmedium und/oder das Hefeerzeugnis können dem Brauprozess entnommen werden. Insbesondere kann das erste Nährmedium einer Ausschlagwürze oder Anstellwürze sein. Ferner kann das Hefeerzeugnis eine Reinzuchthefe, beispielsweise angezogen auf gehopfter Würze, oder eine Erntehefe, beispielsweise aus der ersten, zweiten oder einer höheren Führung, sein. Für diese Fälle haben die Erfinder herausgefunden, dass im ersten Nährmedium und/oder im Hefeerzeugnis offenbar Hemmstoffe enthalten sein können, die für die Erzeugung von Vitamin B₁₂ mittels den vorgeschlagenen Milchsäurebakterienarten abträglich sein können. Wahrscheinlich handelt es hierbei um die Hopfenbitterstoffe.

Nach Erkenntnis der Erfinder können, beispielsweise durch ein- oder mehrmaliges Waschen des Hefeerzeugnisses mit Wasser, insbesondere Trinkwasser oder Brauwasser, die Hopfenbitterstoffe vollständig oder im Wesentlichen vollständig aus dem Hefeerzeugnis entfernt werden. Der Einsatz eines derartigen, von Hopfenbitterstoffen befreiten Hefeerzeugnisses im erfindungsgemäßen Verfahren führt zu einer Steigerung der Vitamin B₁₂-Erzeugung.

In ähnlicher Weise konnte eine wenigstens teilweise Hemmung der Vitamin B₁₂-Erzeugung im erfindungsgemäßen Verfahren festgestellt werden, wenn das erste Nährmedium nicht frei oder nicht im Wesentlichen frei von Hopfenbitterstoffen war. Vorteilhaft ist daher der Einsatz einer Maische oder ungehopften Würze, beispielsweise einer Vorderwürze oder ungehopften Ausschlag- oder Anstellwürze, als erstes Nährmedium, welche frei von Hopfenbitterstoffen sind.

Ferner kann das Hefeerzeugnis aus einer obergärigen oder untergärigen Hefe der Gattung *Saccharomyces,* insbesondere der Art *Saccharomyces cerevisiae* oder der Art *Saccharomyces carlsbergensis,* gewonnen werden. Dabei ist die Hefe vorzugsweise eine Reinzuchthefe oder eine Erntehefe aus dem Bierbereitungsverfahren. Ferner werden die Hopfenbitterstoffe aus der Hefe oder dem Hefeerzeugnis vollständig oder im Wesentlichen vollständig entfernt, vorzugsweise durch ein- oder mehrmaliges Waschen der Hefe oder des Hefeerzeugnisses mit Wasser, insbesondere mit Trinkwasser oder Brauwasser.

Indem das Hefeerzeugnis aus einer brauereiüblichen, obergärigen oder untergärigen Hefe gewonnen wird, insbesondere aus einer Erntehefe, steht im Falle der Durchführung des erfindungsgemäßen Verfahrens in einer Brauerei eine kostengünstige und mengenmäßig praktisch unbegrenzte Quelle für den Rohstoff des Hefeerzeugnisses zur Verfügung.

Darüber hinaus haben Untersuchungen gezeigt, dass ein unter Verwendung der genannten Hefen gewonnenes Hefeerzeugnis sich besonders positiv auf die Vitamin B₁₂-Erzeugung gemäß dem erfindungsgemäßen Verfahren auswirkt.

Zudem kann das Verfahren ferner die Schritte aufweisen:
(e) Bereitstellen eines zweiten Nährmediums, vorzugweise einer Maische oder einer Würze, insbesondere einer Ausschlagwürze; und
(f) Mischen des in Schritt (b) erhaltenen Mediums mit dem zweiten Nährmedium.

Durch das Mischen des in Schritt (b) erhaltenen Mediums mit einem zweiten Nährmedium, vorzugsweise mit einer Maische oder einer Würze, kann durch einen einfachen, weiteren Verfahrensschritt eine biologisch gesäuerte Maische oder Würze erzeugt werden. Die technologischen Vorteile der biologischen Maische- oder Würzesäuerung sind dem Fachmann bekannt. Über die herkömmlichen Vorteile hinaus erlaubt das erfindungsgemäße Verfahren zudem die Gewinnung einer Maische oder Würze mit einem erhöhten Gehalt an bioverfügbarem Vitamin B₁₂.

Wenn eine Maische oder Würze als zweites Nährmedium verwendet wird, wird vorteilhaft erzielt, dass das erzeugte Vitamin B₁₂ quantitativ im Wesentlichen erhalten bleibt und nicht von den Milchsäurebakterien wieder aufgenommen oder verstoffwechselt wird. Darüber hinaus bleibt das Vitamin B₁₂ im Wesentlichen vollständig oder wenigstens zu einem hohen Anteil bioverfügbar. Damit läßt sich eine Maische oder Würze erzeugen, die einen gegenüber herkömmlichen Maischen oder Würzen erheblich erhöhten Gehalt an Vitamin B₁₂ aufweist, ohne übersäuert zu sein.

So kann eine erfindungsgemäß erzeugte Maische einen pH-Wert im Bereich von 4,5 bis 5,7, vorzugsweise 4,9 bis 5,3, aufweisen. Eine erfindungsgemäß erzeugte Würze kann einen pH-Wert im Bereich von 4,2 bis 5,7, vorzugsweise 4,6 bis 5,0, aufweisen.

Die Einstellung des pH-Werts der Maische oder Würze auf die angegebenen Werte führt zu technologischen Vorteilen, wie beispielsweise verbesserte Geschmacksstabilität, Schaumstabilität und hellere Bierfarbe.

Das Verfahren kann ferner die Schritte aufweisen:
(i) vorzugsweise Läutern des in Schritt (b) oder (f) erhaltenen Mediums,
(k) Kochen oder Heißhalten und vorzugsweise Hopfung des in Schritt (f) oder (i) erhaltenen Mediums;
(1) wenigstens teilweises Entfernen von Trub aus dem in Schritt (k) erhaltenen Medium; und
(m) vorzugsweise Einstellen der Temperatur des in Schritt (1) erhaltenen Mediums auf eine Anstelltemperatur.

Durch die Anwendung der Schritte (i) bis (m) lässt sich die erfindungsgemäße, biologisch gesäuerte Maische oder Würze zu einer vergärbaren Ausschlag- und Anstellwürze weiterverarbeiten, welche ebenfalls einen erhöhten Gehalt an bioverfügbarem Vitamin B₁₂ aufweist.

So kann das Verfahren ferner den Schritt aufweisen:
(p) Verarbeiten des in einem der Schritte (b), (f), (k), (1) oder (m) erhaltenen Mediums zu einem Getränk, vorzugsweise Behandeln dieses Mediums mit einer Hefe der Gattung *Saccharomyces,* insbesondere mit der Art *Saccharomyces cerevisiae* oder der Art *Saccharomyces carlsbergensis.*

Die in den verschiedenen Verfahrensschritten gewonnenen Vorstufen lassen sich erfindungsgemäß auf herkömmliche Weise, beispielsweise durch alkoholische und nicht-alkoholische Gärung, zu einem Getränk weiterverarbeiten, welches ebenfalls einen erhöhten Gehalt an bioverfügbarem Vitamin B₁₂ aufweist. Dieses Getränk kann insbesondere sein: ein alkoholfreies Getränks, ein Bier, insbesondere ein alkoholfreies Bier, ein Weizenbier, insbesondere ein alkoholfreies Weizenbier, ein bierhaltiges Getränk, insbesondere ein Biermischgetränk, ein mit einer Hefe fermentiertes oder nicht mit Hefe fermentiertes Getränk. Das erfindungsgemäße Getränk weist einen erhöhten Gehalt an bioverfügbarem Vitamin B₁₂ auf.

Trotz des erfindungsgemäß weiter gesteigerten Gehalts an Vitamin B₁₂ im fertigen Getränk findet nach Erkenntnis der Erfinder eine Beeinträchtigung der Bioverfügbarkeit bzw. einer Abreicherung in den weiteren Verfahrensschritten bis zum fertigen Getränk nicht oder im Wesentlichen nicht statt. Damit gelten dieselben Vorteile des erfindungsgemäß behandelten ersten Nährmediums, insbesondere des erzeugten Sauergutes, analog auch für das erfindungsgemäß hergestellte Getränk, insbesondere für ein Bier und ein alkoholfreies Bier.

Das Verfahren kann ferner den Schritt aufweisen:
(q) Verarbeiten des in einem der Schritte (b), (f), (k), (1) oder (m) erhaltenen Mediums oder des in Schritt (p) erhaltenen Getränks zu einem nicht fluiden, insbesondere festen, Nahrungsmittel;
   wobei das in einem der Schritte (b), (f), (k), (1) oder (m) erhaltene Medium oder das in Schritt (p) erhaltene Getränk mit einer Vorstufe des nicht fluiden Nahrungsmittels gemischt wird.

Die in den verschiedenen Verfahrensschritten gewonnenen Vorstufen oder das Getränk lassen sich erfindungsgemäß auf herkömmliche Weise, beispielsweise durch Einengen und/oder Mischen mit anderen Bestandteilen, zu einem nicht fluiden Nahrungsmittel weiterverarbeiten, welches ebenfalls einen erhöhten Gehalt an bioverfügbarem Vitamin B₁₂ aufweist. Dieses Nahrungsmittel kann insbesondere sein: ein getreidehaltiges Nahrungsmittel, insbesondere ein getreidehaltiger Riegel, Frühstückscerealien, ein Malzextraktprodukt, eine Backware, ein Milchprodukt, insbesondere ein Joghurt.

Damit gelten die Vorteile des erfindungsgemäß hergestellten Getränks für das vorstehend erläuterte, erfindungsgemäße nicht fluide Nahrungsmittel analog.

Der Massenanteil an Wasser im Medium, welches in einem der Schritte (b), (f), (k), (1) oder (m) erhalten wurde, im in Schritt (p) erhaltenen Getränk oder im in Schritt (q) erhaltenen, nicht fluiden Nahrungsmittel kann auf weniger als 35 %, vorzugsweise weniger als 30 %, vorzugsweise weniger als 25 %, vorzugsweise auf weniger als 20 %, insbesondere auf weniger als 15 %, eingestellt werden.

Höhere als die vorstehend angegebenen Wasseranteile erlauben nicht die Sicherstellung der mikrobiologischen Stabilität des mit dem erfindungsgemäßen Verfahren erzeugten Nahrungsmittels oder einer Vorstufe desselben. Wird der Wassergehalt durch Wasserentzug eingestellt, werden aufgrund der Volumenverringerung die Transport-und Lagerkosten für das konzentrierte Medium verringert.

Darüber hinaus kann das konzentrierte Medium überall und unabhängig vom Herstellungsort durch Rückverdünnung auf die Ursprungskonzentration oder eine gewünschte Konzentration konfektioniert werden.

Ferner weist das konzentrierte Medium eine gegenüber dem Ausgangsstoff erhöhte Viskosität auf, die bei der Verwendung des konzentrierten Mediums zur Herstellung von Nahrungsmitteln vorteilhaft ist. So kann das konzentrierte Medium beispielsweise als Bindemittel für körnige oder pulverige Nahrungsmittelbestandteile dienen.

Ferner ist es vorteilhaft, wenn der Massenanteil an Wasser im erfindungsgemäß erzeugten Medium oder Getränk auf mehr als 0 %, insbesondere mehr als 5 %, eingestellt wird.

Durch das Einstellen eines definierten Restwassergehaltes wird die Staubbildung bei der Verarbeitung oder Handhabung des Nahrungsmittels oder der Vorstufen desselben vermieden.

Die erfindungsgemäße Aufgabe wird ferner durch den Gegenstand des Erzeugnisanspruchs 9 gelöst. Darin wird ein Nahrungsmittel oder eine Vorstufe desselben beansprucht, das mit einem Verfahren nach einem der Ansprüche 1 bis 8 hergestellt wird.

Dabei gelten die bei der Beschreibung des erfindungsgemäßen Herstellungsverfahrens genannten Vorteile für ein mittels dieses Verfahrens erzeugtes Nahrungsmittel oder eine Vorstufe desselben, insbesondere ein Sauergut, eine Maische, eine Würze, ein Getränk oder ein nicht fluides Nahrungsmittel, analog.

Die erfindungsgemäße Aufgabe wird ferner durch den Gegenstand der Verwendungsansprüche 10 und 11 gelöst.

Darin wird die Verwendung eines Hefeerzeugnisses in einem Verfahren zur Herstellung eines Nahrungsmittels oder einer Vorstufe desselben, vorzugsweise in einem Verfahren nach einem der Ansprüche 1 bis 8, beansprucht. Dabei weist das Verfahren wenigstens die Schritte auf:
(a) Bereitstellen eines ersten Nährmediums, vorzugsweise einer Maische oder einer Würze, insbesondere einer Vorderwürze, oder eines Glattwassers; und
(b) Behandeln des ersten Nährmediums mit Milchsäurebakterien der Art *Lactobacillus coryniformis,* insbesondere der Unterart *Lactobacillus coryniformis* subsp. *coryniformis* (DSMZ Nr. 20007), der Art *Lactobacillus backii* (DSMZ Nr. 18080), der Art *Lactobacillus plantarum* (vorzugsweise DSMZ Nr. 2601 oder 2648 oder 13273), insbesondere der Unterarten *Lactobacillus plantarum* subsp. *plantarum* (DSMZ Nr. 20174) oder *Lactobacillus plantarum* subsp. *argentoratensis* (DSMZ Nr. 16365), oder der Art *Lactobacillus fermentum* (DSMZ Nr. 20052) oder mit einem Gemisch aus Milchsäurebakterien von wenigstens zwei dieser Arten oder Unterarten.

Dabei findet die Behandlung des Schrittes (b) in Anwesenheit des Hefeerzeugnisses statt. Ferner enthält das Hefeerzeugnis ein Extrakt, ein Autolysat und/oder eine getrocknete Form einer Hefe. Das Hefeerzeugnis kann auch aus einem Extrakt, einem Autolysat und/oder einer getrockneten Form einer Hefe bestehen.

Ferner wird die Verwendung eines Verfahrens zur Herstellung eines Nahrungsmittels oder einer Vorstufe desselben nach einem der Ansprüche 1 bis 8 zur Steigerung der Bioverfügbarkeit und/oder der erzeugten Menge und/oder der Massenkonzentration von Vitamin B₁₂, vorzugsweise von bioverfügbarem Vitamin B₁₂, im Nahrungsmittel oder in der Vorstufe desselben beansprucht.

Die bei der Beschreibung des erfindungsgemäßen Herstellungsverfahrens genannten Vorteile gelten für die erfindungsgemäßen Verwendungen einschließlich deren spezifizierten Ausführungsformen analog. Zudem sind alle in Zusammenhang mit dem erfindungsgemäßen Verfahren offenbarten Merkmale auch mit der erfindungsgemäßen Verwendung kombinierbar.

Dabei kann das Hefeerzeugnis in einer Massenkonzentration im Bereich von ≥ 0,2 und ≤ 20 g/l bezogen auf das erste Nährmedium vorliegen.

Zudem kann das erste Nährmedium frei oder im Wesentlichen frei von Hopfenbitterstoffen sein. Zusätzlich oder alternativ kann das Hefeerzeugnis frei oder im Wesentlichen frei von Hopfenbitterstoffen sein.

### Alternativen und weitere Offenbarung der Erfindung

Die Erfindung ist nicht auf die Verwendung von *Lactobacillus coryniformis, insbesondere Lactobacillus coryniformis* subsp. *coryniformis,* beschränkt. Die vorstehend beschriebenen Herstellungsverfahren sowie Erzeugnisse lassen sich alternativ auch mit Milchsäurebakterien der Arten *Lactobacillus backii, Lactobacillus plantarum* oder *Lactobacillus fermentum* oder mit einem Gemisch aus Milchsäurebakterien von wenigstens zwei dieser Arten oder der offenbarten Unterarten durchführen bzw. herstellen. Hierdurch werden die vorstehend beschriebenen Vorteile und Eigenschaften in analoger Weise verwirklicht.

Erfindungsgemäß kann die Massenkonzentration bzw. der Gehalt des Hefeerzeugnisses bezogen auf das erste Nährmedium auch auf den Bereich von mehr als 0,2 bis weniger als 10 g/l beschränkt sein. Mit der Wahl der oberen Grenze von 10 g/l wird eine Sedimentbildung im Redaktionsgefäß zuverlässig vermieden.

Zudem kann die Massenkonzentration bzw. der Gehalt des Hefeerzeugnisses bezogen auf das erste Nährmedium, insbesondere bei der getrockneten Form der Hefe, auch auf den Bereich von mehr als 5 bis weniger als 10 g/l beschränkt sein. In diesem Konzentrationsbereich wird eine hohe Steigerung der Vitamin B₁₂-Erzeugung erzielt, wobei die vorstehend diskutierten Nachteile von erhöhten Hefe-Konzentrationen nicht oder nur minimal auftreten.

Wird als Hefeerzeugnis ein Hefeextrakt oder ein Hefeautolysat gewählt, so kann dessen Massenkonzentration im Bereich von mehr als 3 bis weniger als 15 g/l gewählt werden, um eine hohe Steigerung der Vitamin B₁₂-Erzeugung und gleichzeitig eine akzeptable Qualität des resultierenden Nahrungsmittels oder seiner Vorstufen zu erreichen.

Ferner kann in diesem Fall der Massenkonzentrationsbereich auf mehr als 0,2 bis weniger als 4 g/l beschränkt werden. In diesem Konzentrationsbereich tritt bereits eine signifikante Steigerung der Vitamin B₁₂-Erzeugung auf, wobei die nachteiligen Erscheinungen der hohen Massenkonzentrationsbereiche vollständig ausbleiben. In diesem Bereich ist die Erzeugung von Vitamin B₁₂ zudem besonders wirtschaftlich.

Andererseits kann für ein Hefeextrakt oder ein Hefeautolysat auch ein Massenkonzentrationsbereich von mehr als 4 bis weniger als 10 g/l gewählt werden. In diesem Bereich fällt die Steigerung der Vitamin B₁₂-Erzeugung besonders hoch aus, während die negativen Begleiterscheinungen aufgrund der hohen Hefeerzeugnis-Konzentration - wenn überhaupt - nur minimal auftreten. Damit kann in diesem Bereich eine hohe Vitamin B₁₂-Ausbeute bei akzeptabler Geruchs- bzw. Geschmacksqualität des resultierenden Nahrungsmittels oder seiner Vorstufen erzielt werden.

Vorteilhaft kann eine Vorstufe des Hefeerzeugnisses, insbesondere eine obergärige oder untergärige Reinzuchthefe oder Erntehefe aus der Brauerei, mit Wasser oder einem anderen geeigneten Stoff ein- oder mehrfach gewaschen werden. Dies geschieht beispielsweise durch Suspendieren der Hefezellen im Wasser und Abzentrifugieren. Nach Verwerfen des Überstandes kann die derart gewaschene Hefemasse erneut in Wasser suspendiert und anschließend wieder abzentrifugiert werden. Die Schritte des Resuspendierens und des Abzentrifugierens können so oft wiederholt werden, bis ein gewünschter Reinheitsgrad erreicht ist, insbesondere bis die Hefe frei oder im Wesentlichen frei von Hopfenbitterstoffen ist.

Vorteilhaft wird als erstes Nährmedium eine Vorderwürze aus der Brauerei gewählt, wobei die Vorderwürze in Schritt (a) einen Extraktgehalt im Bereich von 7 bis 28 %, vorzugsweise 10 bis 22 %, vorzugsweise 12 bis 19 %, insbesondere 14 bis 16 %, aufweist.

Vorderwürze als erstes Nährmedium zeichnet sich durch einen hohen Gehalt an für die im erfindungsgemäßen Verfahren verwendeten Milchsäurebakterien erforderlichen Nährstoffen aus. Darüber hinaus ist Vorderwürze gut verfügbar und mit den Anlagen einer herkömmlichen Brauerei einfach zu erzeugen.

Zudem kann eine große Konzentrationsbreite hinsichtlich des Extraktgehaltes der Vorderwürze eingesetzt werden, wodurch das Verfahren in der Anwendung flexibel ist.

Das erfindungsgemäße Verfahren ist auch bei hochkonzentrierten Nährmedien, welche beispielsweise beim High-Gravity-Verfahren gewonnen werden, unter Erzielung der damit verbundenen Vorteile, insbesondere Volumen- und Kostenersparnis, anwendbar.

In einer vorteilhaften Ausführungsform wird das erste Nährmediums vor dem Behandeln mit den Milchsäurebakterien mit Wasser, insbesondere Brauwasser, derart verdünnt, dass der Extraktgehalt der resultierenden Verdünnung im Bereich von 5 bis 10 %, vorzugsweise 6 bis 9 %, insbesondere 7 bis 8 %, beträgt.

Durch eine Verdünnung des ersten Nährmediums kann die für die im erfindungsgemäßen Verfahren verwendeten eingesetzten Mikroorganismen optimale Nährstoffkonzentration eingestellt werden.

Zudem kann der Verbrauch an dem zu erzeugenden ersten Nährmedium verringert werden.

Das erste Nährmedium kann mit den Milchsäurebakterien in einer Menge angeimpft werden, so dass die optische Dichte (OD) des ersten Nährmediums unmittelbar nach dem Animpfen im Bereich von etwa 0,1 bis 1,0 OD, vorzugsweise 0,2 bis 0,8, vorzugsweise 0,3 bis 0,7, vorzugsweise 0,4 bis 0,5 OD, liegt, insbesondere etwa 0,45 OD beträgt, wobei der Messwert der optischen Dichte bei einer Wellenlänge von 620 nm gemessen wird und um den Einfluss des ersten Nährmediums bereinigt ist.

Das Animpfen des ersten Nährmediums mit den erfindungsgemäß verwendeten Mikroorganismen derart, dass die vorstehend erwähnte optische Dichte (OD) oder eine entsprechende Zelldichte zu Beginn der Behandlung eingestellt wird, führt zu einer schnellen Umsetzung und damit zu einer vorteilhaften kurzen Behandlungszeit.

Darüber hinaus führt eine optimale Animpfkonzentration der Milchsäurebakterien zu einem optimalen Aromaprofil mit minimalen Konzentrationen an Fehlaromen oder deren vollständiger Abwesenheit.

Eine optische Dichte des ersten Nährmediums unmittelbar nach dem Animpfen und gegebenenfalls Homogenisieren von weniger als 0,4, vorzugsweise 0,3, vorzugsweise 0,2 und insbesondere 0,1 erfordert nachteilig eine zu lange Zeitdauer bis eine angestrebte, hohe Stoffumsatzrate der Milchsäurebakterien erreicht ist.

Dagegen bewirkt eine optische Dichte des ersten Nährmediums unmittelbar nach dem Animpfen und gegebenenfalls Homogenisieren von mehr als 0,5, vorzugsweise 0,7, vorzugsweise 0,8, insbesondere 1,0 nicht optimale Wachstumsbedingungen für die Milchsäurebakterien, beispielsweise wegen Feedback-Hemmung.

Die Milchsäurebakterien können sich zu Beginn der Behandlung des ersten Nährmediums, insbesondere bei deren Zugabe zum ersten Nährmedium, gemäß Schritt (b) in der log-Phase bzw. Wachstumsphase befinden.

Ein Animpfen mit Mikroorganismen, welche sich in der log-Phase (logarithmische Phase) bzw. Wachstumsphase befinden, führt aufgrund der hohen Aktivität der eingesetzten Mikroorganismen vorteilhaft zu einer schnellen Umsetzung des ersten Nährmediums zum Sauergut.

Die Dauer der Behandlung gemäß Schritt (b) kann zwischen etwa 5 und 50 Stunden, vorzugsweise 20 bis 48, insbesondere 30 bis 44 Stunden, insbesondere 36 bis 42 Stunden, betragen.

Der erfindungsgemäß vorgesehene Behandlungszeitraum kann vorteilhaft auf die vorstehend angegebenen, kurzen Zeitdauern beschränkt werden. Hierdurch kann eine kurzfristige Bereitstellung eines behandelten ersten Nährmediums, insbesondere eines Sauerguts, realisiert werden. Insbesondere ist bei einer Behandlungsdauer von weniger als 5 Stunden die Ausbeute an Vitamin B₁₂ und/oder Laktat zu gering. Dagegen wird bei einer Behandlungsdauer von mehr als 50 Stunden keine weitere Steigerung der Erzeugung von bioverfügbarem Vitamin B₁₂ erzielt. Ferner besteht die Gefahr der Übersäuerung.

Die Behandlung gemäß Schritt (b) kann bei einer Temperatur des Nährmediums im Bereich von etwa 15 bis 48 °C, vorzugweise 25 bis 42 °C, insbesondere 32 bis 40 °C, insbesondere 35 bis 39 °C, insbesondere 36 bis 38 °C, durchgeführt werden.

Die Behandlung gemäß Schritt (b) kann vorteilhaft in einem breiten Temperaturbereich erfolgen. Eine Auswahl der Temperatur zwischen etwa 30 und 40 °C schafft optimale Wachstumsbedingungen für die verwendeten Mikroorganismen, wodurch sich die erforderliche Behandlungszeit verkürzt und eine optimale Qualität des resultierenden Erzeugnisses erhalten wird.

Das Verfahren kann ferner einen Schritt aufweisen, in dem das gemäß Schritt (b) behandelte erste Nährmedium sterilisiert wird.

Durch einen abschließenden Sterilisationsschritt kann ausgeschlossen werden, dass die erfindungsgemäß eingesetzten Milchsäurebakterien bei der weiteren Verwendung des Erzeugnisses, beispielsweise im Rahmen einer Nahrungsmittel- oder Getränkeherstellung, insbesondere bei der Bierherstellung, einen unerwünschten Einfluss auf das jeweilige Erzeugnis haben können.

So wird insbesondere bei einem Einsatz von Hefe in einem späteren Verfahrensschritt deren Stoffwechselaktivitäten nicht negativ beeinflusst.

Die Sterilisation kann damit mit allen dem Fachmann bekannten Mittel erfolgen. Bevorzugt ist dabei die Zugabe des Sauerguts zur kochenden oder heißen Würze.

Das in einem der Schritte (b), (f), (k), (1) oder (m) erhaltene Medium, das in Schritt (p) erhaltene Getränk oder das in Schritt (q) erhaltene, nicht fluide Nahrungsmittel kann einen Massenanteil von Milchsäure im Bereich von etwa 0,1 bis 1,0 %, vorzugweise 0,2 bis 0,6 %, vorzugweise etwa 0,3 bis 0,5 %, insbesondere etwa 0,35 bis 0,45 %, aufweisen.

Durch das Vorhandensein von Milchsäure in den angegebenen Massenanteilen lässt sich das erfindungsgemäß erzeugte Medium, insbesondere das Sauergut, vorteilhaft zur Einstellung eines bestimmten pH-Wertes, beispielsweise von Maische oder Würze, verwenden. Hierdurch lässt sich auf natürliche Weise und entsprechend dem Reinheitsgebot eine pH-Wert-Einstellung von Nahrungsmitteln oder entsprechenden Vorstufen derselben, insbesondere von Maische oder Würze, bewerkstelligen.

Das zweite Nährmedium kann bei Durchführung des Schritts (f) eine Temperatur von wenigstens 50 °C, vorzugsweise wenigstens 60 °C, vorzugsweise wenigstens 70 °C, vorzugsweise wenigstens 80 °C, insbesondere wenigstens 90 °C, insbesondere wenigstens 95 °C, aufweisen.

Durch die Zugabe des in Schritt (b) erhaltenen Mediums zum zweiten Nährmedium bei einer hohen Temperatur wird eine wirksame Inaktivierung bzw. Sterilisation wenigstens eines Teils der Milchsäurebakterien in einem Schritt erzielt. Hierdurch entfällt ein gesonderter Sterilisationsschritt sowie der damit verbundene Kosten-, Zeit- und Energieaufwand.

Das in Schritt (b) erhaltene Medium kann in einem Volumenanteil von 2 bis 20 %, vorzugsweise von 5 bis 15 %, vorzugsweise 6 bis 12 %, vorzugsweise 7 bis 11%, insbesondere 8 bis 10 %, bezogen auf das Volumen der resultierenden Mischung, zugegeben werden.

Durch Einstellung eines derartigen Volumenanteils des Sauerguts an der resultierenden Mischung kann ein für die Maische bzw. Würze optimaler pH-Wert, vorzugsweise ein pH-Wert der resultierenden Mischung im Bereich von 4,2 bis 5,5, vorzugsweise 4,6 bis 5,3, erzielt werden.

Das Behandeln des ersten Nährmediums mit Milchsäurebakterien gemäß Schritt (b) kann unter im Wesentlichen anaeroben Bedingungen, insbesondere unter anaeroben Bedingungen, stattfinden.

Möglicherweise hat die Wahl von anaeroben Bedingungen bzw. im Wesentlichen anaeroben Bedingungen während der Behandlung des Nährmediums mit den erfindungsgemäß eingesetzten Milchsäurebakterien eine positive Auswirkung auf Quantität und/oder Bioverfügbarkeit des erzeugten Vitamins B₁₂.

Bevorzugt werden anaeroben Bedingungen durch Luftabschluß und/oder Begasen mit CO₂ oder N₂ oder anderen bekannten Maßnahmen hergestellt.

Das erfindungsgemäß erzeugte Nahrungsmittel, insbesondere das Getränk, kann konform mit dem deutschen Reinheitsgebot sein oder ausschließlich aus Zutaten hergestellt werden, welche nach dem deutschen Reinheitsgebot für die Herstellung von Bier zugelassen sind. So können die Rohstoffe des erfindungsgemäßen Nahrungsmittels bzw. Getränks auf die gemäß dem deutschen Reinheitsgebot zugelassenen Rohstoffe zum Bierbrauen, insbesondere Gerstenmalz, Weizenmalz und Brauwasser sowie die erfindungsgemäß eingesetzten Mikroorganismen, beschränkt werden. Damit wird es erfindungsgemäß erstmalig möglich, ein Nahrungsmittel, insbesondere ein Getränk, mit bioverfügbarem Vitamin B₁₂ in erhöhter Massenkonzentration bereitzustellen, das dem Reinheitsgebot entspricht.

Das erfindungsgemäß erzeugte Nahrungsmittel kann eine gel- oder pastenartige Konsistenz aufweisen. Eine gel- oder pastenartige Konsistenz begünstigt vorteilhaft eine schnellere bzw. bessere Resorption der darin enthaltenen Nährstoffe, insbesondere des Vitamins B₁₂, im menschlichen oder tierischen Körper. Ferner bewirkt eine gel- oder pastenartige Konsistenz eine verbesserte Verträglichkeit und eine leichte Verzehrbarkeit bzw. Handhabbarkeit, beispielsweise während des Konsums bei Sport- oder Freizeitaktivitäten.

Das erfindungsgemäß erzeugte Nahrungsmittel kann einen Massenanteil an Vitamin B₁₂ von wenigstens 0,15 µg pro Portion, vorzugsweise wenigstens 0,2 µg pro Portion, vorzugsweise wenigstens 0,3 µg pro Portion, vorzugsweise wenigstens 0,35 µg pro Portion, vorzugsweise wenigstens 0,4 µg pro Portion, vorzugsweise wenigstens 0,5 µg pro Portion, vorzugsweise wenigstens 0,6 µg pro Portion, vorzugsweise wenigstens 1,0 µg pro Portion, insbesondere wenigstens 1,5 µg pro Portion des Nahrungsmittels aufweist, wobei eine Portion des Nahrungsmittels eine Masse von 20 g aufweist.

Je höher der Massenanteil an Vitamin B₁₂ im erfindungsgemäßen Nahrungsmittel, desto besser ist die erzielbare Versorgung des menschlichen oder tierischen Körpers mit Vitamin B₁₂.

Das erfindungsgemäß erzeugte Nahrungsmittel kann ein Massenverhältnis von Glucose zu Fructose im Bereich von 1,7:1 bis 2,3:1, vorzugsweise 1,8:1 bis 2,2: 1, vorzugsweise 1,9:1 bis 2,1: 1, insbesondere etwa 2:1, aufweisen. Hierdurch wird die physiologische Wirkung weiter verbessert.

Das erfindungsgemäß erzeugte Nahrungsmittel kann einen Massenanteil an Natriumionen von wenigstens 20 mg pro Portion, vorzugsweise wenigstens 25 mg pro Portion, vorzugsweise wenigstens 30 mg pro Portion, vorzugsweise wenigstens 40 mg pro Portion, insbesondere wenigstens 50 mg pro Portion des Nahrungsmittels aufweisen, wobei eine Portion des Nahrungsmittels eine Masse von 20 g aufweist.

Das erfindungsgemäß erzeugte Nahrungsmittel kann in konzentrierter Form einen Massenanteil an Natriumionen im Bereich von 50 bis 200 mg pro Portion, vorzugsweise 60 bis 150 mg pro Portion, insbesondere 70 bis 100 mg pro Portion, des Nahrungsmittels aufweisen, wobei eine Portion des Nahrungsmittels eine Masse von 20 g aufweist.

Durch Einstellen des erfindungsgemäß vorgesehenen Massenanteils an Natriumionen wird die physiologische Wirkung des Nahrungsmittels, insbesondere die Vorbeugung vor oder Linderung bzw. Vermeidung von Muskelkrämpfen, insbesondere bei Einsatz des erfindungsgemäßen Nahrungsmittels als Sportlernahrung, weiter verbessert. Unterhalb der vorstehend angegebenen Massenanteile an Natriumionen kommt deren physiologische Wirkung nicht hinreichend zur Geltung.

Dabei kann ein Massenanteil an Natriumionen von weniger als 200, vorzugsweise weniger als 55, insbesondere weniger als 50 mg pro Portion gegebenenfalls eine nicht hinreichende physiologische Wirkung aufweisen. Dagegen hat ein Massenanteil an Natriumionen von mehr als 100, vorzugsweise mehr als 120, insbesondere mehr als 135 mg pro Portion gegebenenfalls eine abführende Wirkung beim Konsumenten.

Dem erfindungsgemäßen Nahrungsmittel oder einer seiner Vorstufen können β-Glucan oder β-Glucan-haltige Zusätze zugegeben werden. Das Nahrungsmittel kann β-Glucan in einem Massenanteil von wenigstens 0,25 g pro Portion, vorzugsweise wenigstens 0,3 g pro Portion, insbesondere wenigstens 0,4 g pro Portion des Nahrungsmittels aufweisen, wobei eine Portion des Nahrungsmittels eine Masse von 20 g aufweist.

Die Zugabe von β-Glucan zum erfindungsgemäßen Nahrungsmittel bewirkt aufgrund seiner Eigenschaft als Ballaststoff eine Verbesserung der physiologischen Wirkungen des erfindungsgemäßen Nahrungsmittels, insbesondere für die Ernährung bei sportlich-aktiver Lebensweise.

Das erfindungsgemäß erzeugte Nahrungsmittel kann Getreidebestandteile, vorzugsweise vermälztes und/oder unvermälztes Braugetreide, insbesondere Gerstenmalz und/oder Weizenmalz, enthalten.

Insbesondere bei der Verwendung von Maische oder Würze als erstem Nährmedium, hat die Anmelderin überraschend festgestellt, dass die erfindungsgemäß eingesetzten Milchsäurebakterien in einem derartigen Milieu Vitamin B₁₂ erzeugen und damit zu einer Erzeugung von Vitamin B₁₂ geeignet sind. Ferner wurde überraschend festgestellt, dass das mittels der erfindungsgemäß eingesetzten Milchsäurebakterien erzeugte Vitamin B₁₂ auch wenigstens zu einem hohen Anteil bioverfügbar in Sinne dieser Anmeldung ist. Auch bei der Anwendung des vorstehend definierten, alternativen Verfahrens kann also eine aktive Erzeugung von bioverfügbarem Vitamin B₁₂ erreicht werden, offensichtlich ohne dass ein "Umschalten" des Stoffwechsels der erfindungsgemäß vorgesehenen Milchsäurebakterien auf die Erzeugung von nicht-bioverfügbarem Vitamin B₁₂ stattfindet.

Die in Zusammenhang mit der in dieser Anmeldung beschriebenen Erfindung genannten Merkmale stellen, falls nicht anders erwähnt oder ersichtlich, fakultative Merkmale vorteilhafter Ausführungsformen dar, welche sich beliebig mit den hierin beschriebenen Gegenständen und untereinander kombinieren lassen, soweit der Fachmann hierin kein offensichtliches Hindernis sieht. So lassen sich insbesondere alle für die in dieser Beschreibung angegebenen Merkmale der Verfahren auch mit den in dieser Anmeldung beschriebenen Erzeugnissen verknüpfen, und umgekehrt. Insbesondere sind alle in Zusammenhang mit einem erfindungsgemäßen Erzeugnis genannten Merkmale auf alle weiteren, in dieser Anmeldung beschriebenen Erzeugnissen übertragbar und damit verknüpfbar. Analoges gilt für alle in dieser Anmeldung beschriebenen Verfahren und deren Merkmale. Analoges gilt für die mittels den beschriebenen Merkmalen erzielten Wirkungen und Vorteile.

### Ausführungsbeispiele

### 1. Befreien der Hefe von Hemmstoffen, insbesondere von Hopfenbitterstoffen, durch Waschen

Eine obergärige oder untergärige Reinzuchthefe oder Erntehefe wird mit Brauwasser im Verhältnis 1:9 (50 g Betriebshefe + 400 ml Wasser) suspendiert. Die entstandene Suspension wird 5 min bei 1000 G zentrifugiert. Anschließend wird der Überstand verworfen und das Hefesediment in 150 ml Brauwasser resuspendiert. Die die beiden letzten Schritte können zwei- bis dreimal wiederholt werden.

Die Hefe weist nach dem Waschen einen reinen, frischen, fruchtigen Geruch auf. Es fehlt die ursprünglich vorhandene Bitternote. Im mikroskopischen Präparat erscheinen die Hefezellen intakt. Lediglich im Falle der untergärigen Hefe treten vereinzelt geschädigte Zellen auf. Die gewaschenen Hefezellen weisen neben einem großen, runden Zellkern ein homogenes Plasma auf. Es werden keine Zellwandaufbrüche beobachtet. Nach Vitalfärbung sind die Zellen weiterhin farblos (= lebend).

### 2. Herstellen eines Hefeautolysats

Der Vorgang der Hefeautolyse wird vorzugsweise durch das Aufbrechen der Zellen eingeleitet. Hierfür werden die Hefen mechanisch, thermisch oder chemisch behandelt. Die Autolyse läuft dann während einer mehrstündigen bis mehrtägigen Inkubation der Hefe bei 40 bis 55 °C und bei geeignetem pH-Wert, vorzugsweise bei einem pH-Wert im Bereich von 5 bis 7, ab.

Eine obergärige oder untergärige Reinzuchthefe oder Erntehefe wird in Ausschlagwürze vermehrt. Die Hefe weist einen Trockensubstanzanteil von etwa 16 bis 17 % auf. Sie wird frisch geerntet und nach dem vorstehend beschriebenen Verfahren gewaschen.

Optional können die Hefezellen zum Aufbrechen durch eine oder mehrere der nachfolgenden Maßnahmen vorbehandelt werden:
a) Nasszellaufschluss mittels Hochdruckhomogenisator;
b) Ultraschallbehandlung (mit und ohne Glaskugeln);
c) Vortexen (mit und ohne Glaskugeln); und
d) Zugabe von Propionsäure.

Details zur einzelnen Vorbehandlungsverfahren werden unten erläutert.

Die eigentliche Autolyse der Hefezellen findet dann durch Inkubieren der ggf. vorbehandelten Hefenzellen für 24 Stunden bei ca. 53 °C im Brutschrank (Regelamplitude: 50 bis 55 °C) unter permanenten Rühren des Ansatzes statt. Im Ergebnis entsteht ein flüssiges Autolysat.

### 3. Herstellen eines Hefeextrakts

Das wie vorstehend beschrieben hergestellte, flüssige Autolysat wird durch Wasserentzug aufkonzentriert. Bei Bedarf kann es filtriert und von geschmacklich störenden Stoffen befreit werden.

Die Hauptbestandteile des so gewonnenen Hefeextrakts sind Peptide und Aminosäuren als Ergebnis des Eiweißabbaus sowie Purine und Pyrimidine, die bei der enzymatischen Spaltung der Nukleinsäuren entstehen.

### 4. Details zur Vorbehandlung

### a) Nasszellaufschluss mittels Hochdruckhomogenisator

Der mechanische Aufschluss der Hefezellen wurde mit dem Hochdruckhomogenisator PANDA Plus 2000 der Firma GEA Niro Soavi Deutschland durchgeführt. Der Nasszellaufschluss mittels Hochdruckhomogenisator stellt das bevorzugte Aufschlußverfahren dar.

Als Folge einer speziellen Strömungsdynamik entsteht im verwendeten Homogenisator ein örtlicher statischer Unterdruck (500 bis 1.500 bar, vorzugsweise 800 bis 1.200 bar). Hierdurch kommt es zu einer Blasenbildung sowohl innerhalb der Hefezelle als auch an der Grenzfläche zwischen der Hefezellwand und dem umgebenden Medium (Kavitationseffekt). Wird der Unterdruck später an einem Entspannungsventil spontan wieder abgebaut, so führt das zum Implodieren der Bläschen. Dies zieht eine punktuelle Ruptur der Zellwände nach sich.

Zur Vorbereitung der Hefeproben wird die frisch gezogene Betriebshefe mit Brauwasser verdünnt (1:2, v/v), auf dem Magnetrührer entkohlensäuert und anschließend dreimal gemäß dem vorstehend beschriebenen Verfahren gewaschen.

Jede Hefeprobe durchläuft das beschriebene Aufschlussverfahren zweimal. Die lichtmikroskopische Betrachtung der derart behandelten Zellen ergibt, dass die Zellen nach Anwendung des Homogenisators keinen Protoplasten mehr enthalten und im Präparat nur die Zellhüllen verbleiben.

Die derart aufgeschlossene Hefe wird anschließend bei etwa 53 °C für etwa 24 Stunden im Brutschrank inkubiert und durch die noch intakten Enzyme autolysiert. Um die Autolyse zu stoppen, werden die Ansätze für etwa 30 min gekocht.

Alternativ können zum Zellaufschluß die nachfolgenden Verfahren eingesetzt werden:

### b) Ultraschall (mit und ohne Glaskugeln)

Die Hefeprobe wird mit Brauwasser (10:90, v/v) verdünnt und anschließend mit Ultraschall für 10 Minuten beaufschlagt (Ultraschallbad: MERCK eurolab USR 46 H).

Zur Verstärkung der mechanischen Kräfte können der Hefeprobe während der Ultraschallbehandlung kleine Glasperlen (Fa. Prolabo/VWR, Durchmesser von 2,5 bis 3,5 mm) zugesetzt werden.

### c) Vortexen mittels Reagenzglasschüttler (mit und ohne Glaskugeln)

Die Hefeprobe wird mit Brauwasser (10:90, v/v) verdünnt und anschließend eine Minute lang mittels Reagenzglasschüttler intensiv geschwenkt bzw. gerührt ("Vortexen"; Vortex Genie 2-Schüttler: Bender & Hobein AG, Stufe 8).

Zur Verstärkung der mechanischen Kräfte können der Hefeprobe kleine Glasperlen (Fa. Prolabo/VWR, Durchmesser von 2,5 bis 3,5 mm) während des Vortexens zugesetzt werden.

### d) Zugabe von Propionsäure

Die Hefeprobe wird mit Propionsäure derart versetzt, dass der Anteil der Säure in der Mischung 5 vol.-% beträgt. Der Ansatz wird von Hand und dann mittels Vortexer geschwenkt. Anschließend wird der Ansatz für 15 Minuten über Kopf geschüttelt (TURBULA-Schüttler). Die Proben werden für 2 Stunden bei Raumtemperatur belassen und wiederholt geschwenkt.

### 5. Herstellen von getrockneter Hefe und Hefeflocken

Die Hefesuspension mit einem Trockensubstanzanteil von etwa 15 % wird gleichmäßig auf eine heiße Walze gesprüht (Walzentrockner der Fa. VITAM GmbH, Hameln). Beim Kontakt mit der Walzenoberfläche platzen die Hefezellen. Zellwand und Zellinhalt trocknen an der Walze an und werden nach spätestens 3 Sekunden als Flocken abgeschabt. 101 Hefesuspension liefern etwa 1,5 kg Flocken. Anschließend werden die Hefeflocken im Mörser zerkleinert.

### 6. Versuche mit kommerziell verfügbaren Hefeerzeugnissen

Bei jedem Ansatz wurde 11 einer großtechnisch hergestellten, verdünnten Vorderwürze (50:50, v/v mit Brauwasser), 15 ml einer Bakteriensuspension (L*actobacillus coryniformis* subsp. *coryniformis* (DSMZ Nr. 20007), propagiert in MRS-Bouillon (oder, falls angegeben: einer Vorderwürze) und 4 g eines Hefeerzeugnisses (falls nachstehend nicht anders angegeben) in eine 11-Schottflasche gegeben und homogenisiert (Massenkonzentration des Hefeerzeugnisses: ca. 4 g/l). Der jeweiligen Kontrolle wurde die verdünnte Vorderwürze und die Bakteriensuspension, jedoch kein Hefeerzeugnis zugegeben (soweit nicht anders angegeben). Die Inkubation der Ansätze erfolgte für 24 Stunden bei 37 °C im Brutschrank. Alle Ansätze basieren auf Doppelbestimmungen und wurden mehrfach wiederholt.

Die Mengen an in den vorstehend beschriebenen Ansätzen gebildetem Vitamin B₁₂ (gemessen mit der mikrobiellen Methode von r-Biopharm AOAC-Methode Nr. 101002) sind als Massenkonzentrationen in nachfolgender Tabelle dargestellt:

| Ansatz | kommerzielles Hefeerzeugnis | Vitamin B₁₂-Massenkonzentration |
|---|---|---|
| | | (µg/100 ml) |
| 0 | Kontrolle (ohne Hefeerzeugnis) | 0,32 |
| 1 | Bierhefe trocken (Tabletten) | 0,81 |
| 2 | Bierhefe trocken (Flocken) | 0,99 |
| 3 | Melassehefe trocken (Flocken) | 1,28 |
| 4 | Melassehefe trocken (Flocken) | > 1,80 |
| 5 | Melassehefe trocken (Flocken) | > 1,80 |
| 6 | Hefeextrakt granuliert | 2,56 |

Alle kommerziellen Hefeerzeugnisse induzieren eine verstärkte Bildung von Vitamin B₁₂ gegenüber der Kontrolle, wenn auch in unterschiedlicher Ausprägung. So betrug die Vitamin B₁₂-Erzeugung in Anwesenheit von Hefeerzeugnissen etwa das Zwei- bis Achtfache gegenüber der Kontrolle.

Als Negativkontrolle wurden die Hefeerzeugnisse als solche überprüft: Keines der Hefeerzeugnisse wies eine nachweisbare Mengen an Vitamin B₁₂ auf.

### 7. Versuche mit kommerziell verfügbarem Hefeerzeugnis (Mengenvariation)

In einer weiteren Versuchsreihe wurde der Zusammenhang zwischen der Menge des zugegebenen Hefeerzeugnisses und dem erzeugten Vitamin B₁₂-Gehalt untersucht. Hierzu wurde das Hefeextrakt des Ansatzes 6 (Hefeextrakt granuliert) der vorstehenden Tabelle als Hefeerzeugnis verwendet. Das übrige Versuchsdesign und die Vitamin B₁₂-Bestimmungsmethode waren identisch mit der vorstehend beschriebenen Versuchsreihe.

| Ansatz | Zugabemenge Hefeerzeugnis | Vitamin B₁₂-Massenkonzentration | zusätzliche Vitamin B₁₂-Erzeugung gegenüber Kontrolle | |
|---|---|---|---|---|
| | (g/l) | (µg/100 ml) | (µg/100 ml) | (%) |
| 0 | 0 (Kontrolle) | 0,37 | | |
| 1 | 0,2 | 0,45 | + 0,08 | + 22 % |
| 2 | 0,5 | 0,54 | + 0,17 | + 46 % |
| 3 | 1,0 | 0,86 | + 0,49 | + 132 % |
| 4 | 2,0 | 1,19 | + 0,82 | + 222 % |
| 5 | 4,0 | 1,79 | + 1,42 | + 384 % |

Wie der vorstehenden Tabelle zu entnehmen ist, tritt eine Steigerung der Vitamin B₁₂-Erzeugung gegenüber der Kontrolle um mehr als 20 % bereits bei einer Zugabemenge des Hefeerzeugnisses von 0,2 g/l auf. Die Vitamin B₁₂-Erzeugung nimmt mit zunehmender Zugabemenge wenigstens im hier untersuchten Bereich linear zu (Bestimmtheitsmaß R² = 0,9854).

### 8. Versuche mit Hefeflocken aus Brauereihefe

In einer weiteren Versuchsreihe wurde eine obergärige und untergärige Brauerei-Betriebshefe untersucht. Die frisch geernteten Hefen wurden gemäß dem vorstehend beschriebenen Verfahren durch Waschen von den Hopfenbitterstoffen weitgehend befreit. Die resultierende Hefesuspension, die einen Trockensubstanzanteil von etwa 15 % aufwies, wurde dann mittels Walzentrocknung (maximale Kontaktzeit der Hefezellen mit der Walze: 3 Sekunden) getrocknet. Die trockenen Hefeflocken wurden vor dem Einsatz als Hefeerzeugnis mittels Mörser pulverisiert. Das übrige Versuchsdesign und die Vitamin B₁₂-Bestimmungsmethode waren identisch mit den vorstehend beschriebenen Versuchsreihen.

| Ansatz | Hefeerzeugnis aus Brauereihefe (Flocken; 4 g/l) | Vitamin B₁₂-Massenkonzentration | zusätzliche Vitamin B₁₂-Erzeugung gegenüber Kontrolle |
|---|---|---|---|
| | | (µg/100 ml) | (%) |
| 0 | Kontrolle (ohne Hefeerzeugnis) | 0,29 | |
| 1 | obergärige Hefe | 0,39 | + 34 % |
| 2 | untergärige Hefe | 0,54 | + 86 % |
| 3 | Gemisch aus obergäriger und untergäriger Hefe | 0,51 | + 76 % |

Wie der vorstehenden Tabelle zu entnehmen ist, führt auch die Verwendung einer Brauerei-Betriebshefe zu einer erheblichen Steigerung der Vitamin B₁₂-Erzeugung durch die Milchsäurebakterien. Insbesondere scheint sich die Anwesenheit von untergäriger Hefe positiv auf die Vitamin B₁₂-Erzeugung durch die Milchsäurebakterien auszuwirken.

### 9. Versuche mit Hefeautolysat aus Brauereihefe

In einer weiteren Versuchsreihe wurde aus einer untergärigen und einer obergärigen Brauerei-Betriebshefe jeweils ein Hefeautolysat nach dem vorstehend beschriebenen Verfahren hergestellt. Dazu wurden die frisch geernteten Hefezellen bei 37 °C über 24 Stunden im Brutschrank autolysiert. In einem Teil der Ansätze wurde die beschriebene Autolyse ohne Vorbehandlung durchgeführt. In einem anderen Teil der Ansätze wurden die Zellwände vor Durchführung der Autolyse mittels dem vorstehend beschriebenen Nasszellaufschluss mittels Hochdruckhomogenisator aufgebrochen. Die Vitamin B₁₂-Bestimmungsmethode war identisch mit derjenigen der vorstehend beschriebenen Versuchsreihen. Die Ergebnisse sind nachstehende Tabelle aufgeführt:

| Ansatz | Hefeautolysat aus Brauereihefe | Vitamin B₁₂-Massenkonzentration |
|---|---|---|
| | | (µg/100 ml) |
| 0 | Kontrolle (ohne Hefeautolysat) | 0,152 |
| 1 | Autolysat aus obergäriger Hefe ohne Zellaufbruch | 0,144 |
| 2 | Autolysat aus untergäriger Hefe ohne Zellaufbruch | 0,122 |
| 3 | Autolysat aus obergäriger Hefe mit Zellaufbruch | 0,160 |
| 4 | Autolysat aus untergäriger Hefe mit Zellaufbruch | 0,178 |

Wie aus der vorstehenden Tabelle zu erkennen ist, kann die Vitamin B₁₂-Massenkonzentration bei Einsatz eines Autolysats, das aus einer unbehandelten obergärigen oder untergärigen Brauerei-Betriebshefe stammt, allenfalls bei den Ansätzen leicht gesteigert werden, bei denen die Hefezellen vor dem Autolysieren mechanisch aufgebrochen wurde. Nach Ansicht der Erfinder könnte dieses Ergebnis darauf zurückzuführen sein, dass das aus einer Brauerei-Betriebshefe gewonnene Autolysat Stoffe enthält, welche die bisher beobachtete, steigernde Wirkung von Hefeerzeugnissen auf die Vitamin B₁₂-Erzeugung verhindert. Demzufolge könnte es sein, dass bestimmte Hemmstoffe, vermutlich Hopfenbitterstoffe, im Autolysat vorhanden sind und eine B₁₂-Induktion wirksam hemmen. Diese Hemmstoffe stammen vermutlich aus der gehopften Anstellwürze, mit denen die Hefe während der Vermehrung (Reinzuchthefe) und/oder während der Gärung in Kontakt kommt.

Eine separate Untersuchung der Autolysate zeigte, dass die Vitamin B₁₂-Massenkonzentrationen der Autolysate selber unter den Bedingungen der Ansätze unterhalb der Bestimmungsgrenze (< 0,030 µg/100 ml) lagen. Damit bringen die Autolysate aus Brauereihefe selbst keine nennenswerten Mengen an Vitamin B₁₂ in die Ansätze ein.

Basierend auf diesen Erkenntnissen wurde in einer weiteren Versuchsreihe eine untergärige Brauereihefe gemäß dem vorstehend beschriebenen Verfahren gewaschen, um die Hefe von Hopfenbitterstoffen im Wesentlichen zu befreien. Wie beim vorangegangenen Ansatz wurden die Hefezellen bei 37 °C über 24 Stunden im Brutschrank autolysiert. Die übrigen Bedingungen einschließlich der Vitamin B₁₂-Bestimmungsmethode sind identisch mit denen der vorstehenden Versuchsreihen. Die Ergebnisse sind in nachstehender Tabelle aufgeführt:

| Ansatz | Hefeautolysat aus Brauereihefe | Vitamin B₁₂-Massenkonzentration |
|---|---|---|
| | | (µg/100 ml) |
| 0 | Kontrolle 1 (ohne Milchsäurebakterien und ohne Hefeautolysat) | 0,116 |
| 1 | Kontrolle 2 (mit Milchsäurebakterien und ohne Hefeautolysat) | 0,182 |
| 2 | Autolysat aus gewaschener, untergäriger Hefe | 0,368 |

Die vorstehende Tabelle zeigt, dass durch eine Behandlung des Nährmediums Vorderwürze mit Milchsäurebakterien der Unterart *Lactobacillus coryniformis* subsp. *coryniformis* bei diesem Versuchsdesign eine Vitamin B₁₂-Massenkonzentration erzielt werden kann, die um etwa 57 % über der der Kontrolle (ohne Milchsäurebakterien) liegt (vgl. Ansätze 0 und 1).

Findet die Umsetzung desselben Nährmediums ferner unter den gleichen Bedingungen, jedoch erfindungsgemäß in Anwesenheit eines Autolysats aus gewaschener, untergäriger Hefe statt, so kann die Vitamin B₁₂-Massenkonzentration gegenüber dem Ansatz ohne Hefeautolysat in etwa verdoppelt werden (+ 102 %; vgl. Ansätze 1 und 2).

### 10. Nachweis von bioverfügbarem Vitamin B₁₂

In einer weiteren Versuchsreihe wurde mittels der Nachweismethode "ADVIA Centaur" (111659 Rev. N, 2008-09; VB12 2/12) die Massenkonzentration an IFgebundenem, bioverfügbarem Vitamin B₁₂ in einem erfindungsgemäß hergestellten Sauergut (Vorstufe für Nahrungsmittel) analog zu vorstehendem Punkt 6 gemessen. Die Ergebnisse sind in nachstehender Tabelle aufgeführt:

| Ansatz | Gegenstand | Massenkonzentration an IFgebundenem (bioverfügbarem) Vitamin B₁₂ |
|---|---|---|
| | | (pg/ml) |
| 0 | Kontrolle 1 (Wasser) | 0,000 |
| 1 | Kontrolle 2 (Vorderwürze) | 0,084 |
| 2 | Sauergut (Vorderwürze, umgesetzt mit L. c., ohne Hefeerzeugnis) | 0,146 |
| 3 | Sauergut (Vorderwürze, umgesetzt mit L. c. in Anwesenheit von Hefeextrakt, granuliert, 4 g/l) | 0,499 |
| L. c. = *Lactobacillus coryniformis* subsp. *coryniformis* | | |

Die vorstehende Tabelle zeigt, dass durch eine Behandlung des Nährmediums Vorderwürze mit *Lactobacillus coryniformis* subsp. *coryniformis* bereits eine hohe Massenkonzentration an bioverfügbarem Vitamin B₁₂ im Vergleich zur Kontrolle erzielt werden kann.

Durch die Anwesenheit eines Hefeerzeugnisses wird die Massenkonzentration an bioverfügbarem Vitamin B₁₂ bei ansonsten identischen Versuchsbedingungen weiter gesteigert. Bei dieser Untersuchung erreichte der Ansatz mit granuliertem Hefeextrakt etwa die 3,5-fache der Massenkonzentration des vergleichbaren Kontrollansatzes ohne Hefeerzeugnisses (beide mit Milchsäurebakterien umgesetzt). Gegenüber der Kontrolle ohne Behandlung mit Milchsäurebakterien (nur Vorderwürze) wurde sogar fast die 6-fache Massenkonzentration an bioverfügbarem Vitamin B₁₂ erzielt.

### Vergleich zum Stand der Technik

Herkömmlicherweise werden für die Maische- bzw. Würzesäuerung die Arten *Lactobacillus amylovorus* bzw. *Lactobacillus amylolyticus* verwendet, welche sich zur Erzeugung von entsprechenden Sauerguten und damit angesäuerten Maischen und Würzen langjährig bewährt haben.

So zeichnen sich diese Milchsäurearten durch eine Wachstumsdominanz in Bierwürze wegen Schnellwüchsigkeit aus. Ferner weisen sie ein hohes Säuerungsvermögen aufgrund einer hohen Laktat-Produktion auf. Dies ist auf ihren homofermentativen Stoffwechselcharakter zurückzuführen. Diese Arten wachsen bei hohen Temperaturen (bis 52 °C), so dass sich hohe Vermehrungsraten erzielen lassen.

Darüber hinaus können diese Arten Dextrin und Stärke vergären. Ferner erzeugen sie einen hohen Anteil an L(+)-Laktat. Von erheblicher Bedeutung ist, dass die genannten Milchsäurebakterien nicht bierschädlich sind, da sie hopfenempfindlich sind und bei Temperaturen < 30 °C nicht wachsen können. In der Fachwelt werden *Lactobacillus amylovorus* und *Lactobacillus amylolyticus* auch deshalb als geeignete Säuerungsorganismen angesehen, weil sie keine Amine (Histamin) oder andere Toxine ausbilden. Darüber hinaus bilden sie kein Diacetyl oder andere, für Geschmack und Aroma der resultierenden Erzeugnisse nachteiligen Substanzen. Schließlich zeichnen sie sich durch eine leichte Handhabbarkeit in der praktischen Verwendung aus.

Demgegenüber gilt die erfindungsgemäß vorgesehene Art *Lactobacillus coryniformis* in der Fachwelt als Bierschädling. Diese Art wächst in schwach gehopftem Bier und erzeugt Diacetyl, welches zu einem nachteiligen Geschmacksprofil im resultierenden Nahrungsmittel oder Getränk, insbesondere im Bier, führt. Darüber hinaus ist *Lactobacillus coryniformis* in der Lage, bei den typischen Vergärungstemperaturen für Bier, insbesondere für obergäriges Bier, nämlich im Temperaturbereich von 15 bis 48 °C zu wachsen. Weiterhin weist *Lactobacillus coryniformis* gegenüber den herkömmlichen Arten *Lactobacillus amylovorus* und *Lactobacillus amylolyticus* den Nachteil auf, dass diese Art fakultativ heterofermentativ ist, d.h., dessen Säuerungsvermögen ist gegenüber den herkömmlich verwendeten Arten vermindert. Folglich muss in etwa die doppelte Menge an Sauergut im Vergleich zu herkömmlichen Arten eingesetzt werden. Hierdurch werden größere Produktionsanlagen erforderlich, und die mit der Säuerung verbundenen Kosten steigen.

Die vorstehend genannte Mehrzahl von Nachteilen sowie die dem Fachmann darüber hinaus bekannten Nachteile der hier betrachteten Arten *Lactobacillus coryniformis, Lactobacillus backii, Lactobacillus plantarum* und *Lactobacillus fermentum* bedeuteten bisher ein wesentliches Hindernis für den Einsatz dieser Arten oder zugehöriger Unterarten in der Getränke- und Nahrungsmittel-herstellenden Industrie, insbesondere in Brauereien und Mälzereien.

## Patentansprüche

1. Verfahren zur Herstellung eines Nahrungsmittels oder einer Vorstufe desselben, wenigstens mit den Schritten:
(a) Bereitstellen einer Maische oder einer Würze oder eines Glattwassers als ein erstes Nährmedium; und
(b) Behandeln des ersten Nährmediums mit Milchsäurebakterien der Art *Lactobacillus coryniformis,* der Art *Lactobacillus backii* (DSMZ Nr. 18080), der Art *Lactobacillus plantarum* oder der Art *Lactobacillus fermentum* (DSMZ Nr. 20052) oder mit einem Gemisch aus Milchsäurebakterien von wenigstens zwei dieser Arten;
wobei die Behandlung gemäß Schritt (b) in Anwesenheit eines Hefeerzeugnisses stattfindet;
wobei das Hefeerzeugnis ein Extrakt, ein Autolysat und/oder eine getrocknete Form einer Hefe enthält; und
wobei das Hefeerzeugnis vorzugsweise in einer Massenkonzentration im Bereich von ≥ 0,2 und ≤ 20 g/l bezogen auf das erste Nährmedium vorliegt.

2. Verfahren nach Anspruch 1, **dadurch gekennzeichnet, dass** das erste Nährmedium frei oder im Wesentlichen frei von Hopfenbitterstoffen ist; und/oder
das Hefeerzeugnis frei oder im Wesentlichen frei von Hopfenbitterstoffen ist; wobei "frei von Hopfenbitterstoffen" in Bezug auf das erste Nährmedium die vollständige Abwesenheit von Hopfenbitterstoffen im ersten Nährmedium bedeutet; wobei "frei von Hopfenbitterstoffen" in Bezug auf das Hefeerzeugnis die vollständige Abwesenheit von Hopfenbitterstoffen im Hefeerzeugnis bedeutet;
wobei "im Wesentlichen frei von Hopfenbitterstoffen" in Bezug auf das erste Nährmedium ein Gehalt an Hopfenbitterstoffen im ersten Nährmedium von höchstens 15 % bezogen auf den Gehalt an Hopfenbitterstoffen, den eine brauereiübliche Anstellwürze für eine Vergärung mit einer untergärigen oder obergärigen Hefe mit Bittereinheiten (EBC-Methode) im Bereich von 15 bis 38 aufweist, bedeutet; und wobei "im Wesentlichen frei von Hopfenbitterstoffen" in Bezug auf das Hefeerzeugnis ein Gehalt an Hopfenbitterstoffen im Hefeerzeugnis von höchstens 20 % bezogen auf den Gehalt an Hopfenbitterstoffen, den eine untergärige oder obergärige Betriebshefe in einer Brauerei aufweist, die während einer Vergärung einer brauereiübliche Würze mit Bittereinheiten (EBC-Methode) im Bereich von 15 bis 38 geerntet wurde, bedeutet.

3. Verfahren nach Anspruch 1 oder 2, **dadurch gekennzeichnet, dass** das Hefeerzeugnis aus einer obergärigen oder untergärigen Hefe der Gattung *Saccharomyces,* insbesondere der Art *Saccharomyces cerevisiae* oder der Art *Saccharomyces carlsbergensis,* gewonnen wird;
wobei die Hefe vorzugsweise eine Reinzuchthefe oder eine Erntehefe aus dem Bierbereitungsverfahren ist;
wobei Hopfenbitterstoffe aus der Hefe oder dem Hefeerzeugnis vollständig oder im Wesentlichen vollständig entfernt werden, vorzugsweise durch ein- oder mehrmaliges Waschen der Hefe oder des Hefeerzeugnisses mit Wasser, insbesondere mit Trinkwasser oder Brauwasser.

4. Verfahren nach einem der Ansprüche 1 bis 3, **dadurch gekennzeichnet, dass** das Verfahren ferner die Schritte aufweist:
(e) Bereitstellen einer Maische oder einer Würze als ein zweites Nährmedium; und
(f) Mischen des in Schritt (b) erhaltenen Mediums mit dem zweiten Nährmedium.

5. Verfahren nach einem der Ansprüche 1 bis 4, **dadurch gekennzeichnet, dass** das Verfahren ferner die Schritte aufweist:
(i) vorzugsweise Läutern des in Schritt (b) oder (f) erhaltenen Mediums,
(k) Kochen oder Heißhalten und vorzugsweise Hopfung des in Schritt (f) oder (i) erhaltenen Mediums;
(1) wenigstens teilweises Entfernen von Trub aus dem in Schritt (k) erhaltenen Medium; und
(m) vorzugsweise Einstellen der Temperatur des in Schritt (1) erhaltenen Mediums auf eine Anstelltemperatur.

6. Verfahren nach einem der Ansprüche 1 bis 5, **dadurch gekennzeichnet, dass** das Verfahren ferner den Schritt aufweist:
(p) Verarbeiten des in einem der Schritte (b), (f), (k), (1) oder (m) erhaltenen Mediums zu einem Getränk, vorzugsweise Behandeln dieses Mediums mit einer Hefe der Gattung *Saccharomyces.*

7. Verfahren nach einem der Ansprüche 1 bis 6, **dadurch gekennzeichnet, dass** das Verfahren ferner den Schritt aufweist:
(q) Verarbeiten des in einem der Schritte (b), (f), (k), (1) oder (m) erhaltenen Mediums oder des in Schritt (p) erhaltenen Getränks zu einem nicht fluiden Nahrungsmittel;
wobei das in einem der Schritte (b), (f), (k), (1) oder (m) erhaltene Medium oder das in Schritt (p) erhaltene Getränk mit einer Vorstufe des nicht fluiden Nahrungsmittels gemischt wird.

8. Verfahren nach einem der Ansprüche 1 bis 7, **dadurch gekennzeichnet, dass** das Verfahren ferner den Schritt aufweist:
(t) Einstellen des Massenanteils an Wasser im Medium, welches in einem der Schritte (b), (f), (k), (1) oder (m) erhalten wurde, im in Schritt (p) erhaltenen Getränk oder des in Schritt (q) erhaltenen, nicht fluiden Nahrungsmittels auf weniger als 35 %, vorzugsweise weniger als 30 %, vorzugsweise weniger als 25 %, vorzugsweise auf weniger als 20 %, insbesondere auf weniger als 15 %; und
vorzugsweise auf mehr als 0 %, insbesondere mehr als 5 %.

9. Nahrungsmittel oder eine Vorstufe desselben, hergestellt mit einem Verfahren nach einem der Ansprüche 1 bis 8.

10. Verwendung eines Hefeerzeugnisses in einem Verfahren zur Herstellung eines Nahrungsmittels oder einer Vorstufe desselben, vorzugsweise in einem Verfahren nach einem der Ansprüche 1 bis 8,
wobei das Verfahren wenigstens die Schritte aufweist:
(a) Bereitstellen einer Maische oder einer Würze oder eines Glattwassers als ein erstes Nährmedium; und
(b) Behandeln des ersten Nährmediums mit Milchsäurebakterien der Art *Lactobacillus coryniformis,* der Art *Lactobacillus backii* (DSMZ Nr. 18080), der Art *Lactobacillus plantarum* oder der Art *Lactobacillus fermentum* (DSMZ Nr. 20052) oder mit einem Gemisch aus Milchsäurebakterien von wenigstens zwei dieser Arten;
wobei die Behandlung des Schrittes (b) in Anwesenheit des Hefeerzeugnisses stattfindet; und
wobei das Hefeerzeugnis ein Extrakt, ein Autolysat und/oder eine getrocknete Form einer Hefe enthält.

11. Verwendung eines Verfahrens zur Herstellung eines Nahrungsmittels oder einer Vorstufe desselben nach einem der Ansprüche 1 bis 8 zur Steigerung der Bioverfügbarkeit und/oder der erzeugten Menge und/oder der Massenkonzentration von Vitamin B₁₂ im Nahrungsmittel oder in der Vorstufe desselben;
wobei "Vitamin B₁₂" auf eine Spezies beschränkt ist, ausgewählt aus einer Gruppe, bestehend aus: Methylcobalamin, Desoxyadenosylcobalamin, Hydroxycobalamin und Sulfitcobalamin.

12. Verwendung nach Anspruch 10 oder 11, **dadurch gekennzeichnet, dass** das Hefeerzeugnis in einer Massenkonzentration im Bereich von ≥ 0,2 und ≤ 20 g/l bezogen auf das erste Nährmedium vorliegt.

13. Verwendung nach einem der Ansprüche 10 bis 12, **dadurch gekennzeichnet, dass** das erste Nährmedium frei oder im Wesentlichen frei von Hopfenbitterstoffen ist; und/oder
das Hefeerzeugnis frei oder im Wesentlichen frei von Hopfenbitterstoffen ist; wobei "frei von Hopfenbitterstoffen" in Bezug auf das erste Nährmedium die vollständige Abwesenheit von Hopfenbitterstoffen im ersten Nährmedium bedeutet; wobei "frei von Hopfenbitterstoffen" in Bezug auf das Hefeerzeugnis die vollständige Abwesenheit von Hopfenbitterstoffen im Hefeerzeugnis bedeutet;
wobei "im Wesentlichen frei von Hopfenbitterstoffen" in Bezug auf das erste Nährmedium ein Gehalt an Hopfenbitterstoffen im ersten Nährmedium von höchstens 15 % bezogen auf den Gehalt an Hopfenbitterstoffen, den eine brauereiübliche Anstellwürze für eine Vergärung mit einer untergärigen oder obergärigen Hefe mit Bittereinheiten (EBC-Methode) im Bereich von 15 bis 38 aufweist, bedeutet; und wobei "im Wesentlichen frei von Hopfenbitterstoffen" in Bezug auf das Hefeerzeugnis ein Gehalt an Hopfenbitterstoffen im Hefeerzeugnis von höchstens 20 % bezogen auf den Gehalt an Hopfenbitterstoffen, den eine untergärige oder obergärige Betriebshefe in einer Brauerei aufweist, die während einer Vergärung einer brauereiübliche Würze mit Bittereinheiten (EBC-Methode) im Bereich von 15 bis 38 geerntet wurde, bedeutet.

## Claims

1. Method for the preparation of a food or a precursor of the same, at least with the steps:
(a) Providing a mash or a wort or last runnings as a first nutrient medium; and
(b) Treating the first nutrient medium with lactic acid bacteria of the species *Lactobacillus coryniformis,* the species *Lactobacillus backii* (DSMZ no. 18080), the species *Lactobacillus plantarum* or the species *Lactobacillus fermentum* (DSMZ no. 20052) or with a mixture of at least two of these species of lactic acid bacteria;
wherein the treatment according to step (b) takes place in the presence of a yeast product;
wherein the yeast product comprises an extract, an autolysate and/or a dried form of a yeast; and
wherein the yeast product is present in a concentration by weight in the range ≥ 0.2 and ≤ 20 g/l based on the first nutrient medium.

2. Method according to claim 1, **characterised in that**
the first nutrient medium is free or essentially free of hop bitter substances; and/or
the yeast product is free or essentially free of hop bitter substances; wherein "free of hop bitter substances" in regard to the first nutrient medium means the total absence of hop bitter substances in the first nutrient medium;
wherein "free of hop bitter substances" in regard to the yeast product means the total absence of hop bitter substances in the yeast product; wherein "essentially free of hop bitter substances" in regard to the first nutrient medium means a content of hop bitter substances in the first nutrient medium of maximum 15 %, based on the content of hop bitter substances which a typical brewery pitching wort has for a fermentation with a bottom-fermenting or top-fermenting yeast with bitter units (EBC Method) in the range 15 to 38; and
wherein "essentially free of hop bitter substances" in regard to the yeast product means a content of hop bitter substances in the yeast product of
maximum 20 %, based on the content of hop bitter substances which a bottom-fermenting or top-fermenting brewery yeast has which was harvested during a fermentation of a typical brewery wort with bitter units (EBC Method) in the range 15 to 38.

3. Method according to claim 1 or 2, **characterised in that** the yeast product is obtained from a top-fermenting or bottom-fermenting yeast of the genus *Saccharomyces,* in particular of the species *Saccharomyces cerevisiae* or of the species *Saccharomyces carlsbergensis;*
wherein the yeast is preferably a pure selected yeast or a harvested yeast from the beer production process;
wherein hop bitter substances are completely or essentially completely removed from the yeast or yeast product, preferably by washing the yeast or yeast product once or more with water, in particular with tap water or brewing water.

4. Method according to one of claims 1 to 3, **characterised in that** the method further has the steps:
(e) providing a mash or a wort as a second nutrient medium;
and
(f) mixing the medium obtained in step (b) with the second nutrient medium.

5. Method according to one of claims 1 to 4, **characterised in that** the method further has the steps:
(i) preferably lautering the medium obtained in step (b) or (f),
(k) mashing out or keeping warm and preferably hopping the medium obtained in step (f) or (i);
(I) at least partly removing the lees from the medium obtained in step (k); and
(m) preferably adjusting the temperature of the medium obtained in step (I) to a pitching temperature.

6. Method according to one of claims 1 to 5, **characterised in that** the method further has the steps:
(p) processing the medium obtained in one of the steps (b), (f), (k), (I) or (m) to a drink, preferably treating this medium with a yeast of the genus *Saccharomyces.*

7. Method according to one of claims 1 to 6, **characterised in that** the method further has the steps:
(q) processing the medium obtained in one of the steps (b), (f), (k), (I) or (m) or the drink obtained in step (p) to a non-fluid food;
wherein the medium obtained in one of the steps (b), (f), (k), (l) or (m) or the drink obtained in step (p) is mixed with a precursor of the non-fluid food.

8. Method according to one of claims 1 to 7, **characterised in that** the method further has the steps:
(t) adjusting the mass fraction of water in the medium that was obtained in one of the steps (b), (f), (k), (I) or (m), in the drink obtained in step (p) or of the non-fluid food obtained in step (q) to less than 35 %, preferably to less than 30 %, preferably to less than 25 %, preferably to less than 20 %, especially to less than 15 %; and
preferably to more than 0 %, especially to more than 5 %.

9. Food or a precursor of the same, produced with a method according to one of claims 1 to 8.

10. Use of a yeast product in a method for the preparation of a food or a precursor of the same, preferably in a method according to one of claims 1 to 8,
wherein the method has at least the steps:
(a) providing a mash or a wort or last runnings as a first nutrient medium; and
(b) treating the first nutrient medium with lactic acid bacteria of the species *Lactobacillus coryniformis,* the species *Lactobacillus backii* (DSMZ no. 18080), the species *Lactobacillus plantarum* or the species *Lactobacillus fermentum* (DSMZ no. 20052) or with a mixture of at least two of these species of lactic acid bacteria;
wherein the treatment of step (b) takes place in the presence of the yeast product; and
wherein the yeast product comprises an extract, an autolysate and/or a dried form of a yeast.

11. Use of a method for preparing a food or a precursor of the same according to one of claims 1 to 8 so as to increase the bio-availability and/or the produced quantity and/or the concentration by weight of vitamin B₁₂ in the food or in the precursor of the same;
wherein "vitamin B₁₂" is limited to a species selected from a group that consists of: methylcobalamin, deoxyadenosylcobalamin, hydroxocobalamin and sulfitocobalamin.

12. Use according to claim 10 or 11, **characterised in that** the yeast product is present in a weight concentration in the range ≥ 0.2 and ≤ 20 g/l based on the first nutrient medium.

13. Use according to one of claims 10 to 12, **characterised in that** the first nutrient medium is free or essentially free of hop bitter substances; and/or
the yeast product is free or essentially free of hop bitter substances; wherein "free of hop bitter substances" in regard to the first nutrient medium means the total absence of hop bitter substances in the first nutrient medium; wherein "free of hop bitter substances" in regard to the yeast product means the total absence of hop bitter substances in the yeast product;
wherein "essentially free of hop bitter substances" in regard to the first nutrient medium means a content of hop bitter substances in the first nutrient medium of maximum 15 %, based on the content of hop bitter substances which a typical brewery wort has for a fermentation with a bottom-fermented or top-fermented yeast with bitter units (EBC Method) in the range 15 to 38; and wherein "essentially free of hop bitter substances" in regard to the yeast product means a content of hop bitter substances in the yeast product of maximum 20 %, based on the content of hop bitter substances which a bottom-fermented or top-fermented
brewery yeast has which was harvested during a fermentation of a typical brewery wort with bitter units (EBC Method) in the range 15 to 38.

## Revendications

1. Procédé servant à fabriquer un aliment ou un précurseur de ce dernier, comprenant au moins les étapes qui suivent consistant à :
(a) fournir une trempe ou un moût ou un dernier lavage en tant que premier milieu nutritif ; et
(b) traiter le premier milieu nutritif avec des bactéries lactiques du type *Lactobacillus coryniformis,* du type *Lactobacillus backii* (DSMZ n° 18080), du type *Lactobacillus plantarum* ou du type *Lactobacillus fermentum* (DSMZ n° 20052) ou avec un mélange de bactéries lactiques d'au moins deux de ces types ;
dans lequel le traitement selon l'étape (b) a lieu en présence d'un produit à base de levure ;
dans lequel le produit à base de levure contient un extrait, un autolysat et/ou une forme séchée d'une levure ; et
dans lequel le produit à base de levure est présent de préférence en une concentration massique située dans la plage ≥ 0,2 et ≤ 20 g/l par rapport au premier milieu nutritif.

2. Procédé selon la revendication 1, **caractérisé en ce que**
le premier milieu nutritif est sans ou sensiblement sans houblon ; et/ou
le produit à base de levure est sans ou sensiblement sans houblon ;
dans lequel « sans houblon » signifie, eu égard au premier milieu nutritif, l'absence totale de houblon dans le premier milieu nutritif ;
dans lequel « sans houblon » signifie, eu égard au produit à base de levure, l'absence totale de houblon dans le produit à base de levure ;
dans lequel « sensiblement sans houblon » signifie, eu égard au premier milieu nutritif, une teneur en houblon dans le premier milieu nutritif de maximum 15 % par rapport à la teneur en houblon, que présente un moût de fermentation utilisé habituellement dans les brasseries en vue d'une fermentation avec une levure à basse fermentation ou à haute fermentation présentant des unités d'amertume (méthode d'analyse de l'EBC [European Brewery Convention]) situées dans la plage allant de 15 à 38 ; et
dans lequel « sensiblement sans houblon » signifie, eu égard au produit à base de levure, une teneur en houblon dans le produit à base de levure de maximum 20 % par rapport à la teneur en houblon, qu'une levure de service à basse fermentation ou à haute fermentation présente dans une brasserie, laquelle a été récoltée au cours d'une fermentation d'un moût habituellement utilisé dans les brasseries présentant des unités d'amertume (méthode d'analyse de l'EBC) situées dans la plage allant de 15 à 38.

3. Procédé selon la revendication 1 ou 2, **caractérisé en ce que**
le produit à base de levure est obtenu à partir d'une levure à haute fermentation ou à basse fermentation du genre *Saccharomyces,* en particulier du genre *Saccharomyces cerevisiae* ou du type *Saccharomyces carlsbergensis ;*
dans lequel la levure est de préférence une levure de souche ou une levure récoltée issue du procédé de préparation de bière ;
dans lequel le houblon est supprimé de la levure ou du produit à base de levure en totalité ou sensiblement en totalité, de préférence par un lavage réalisé une fois ou à plusieurs reprises de la levure ou du produit à base de levure avec de l'eau, en particulier avec de l'eau potable ou de l'eau de brassage.

4. Procédé selon l'une quelconque des revendications 1 à 3, **caractérisé en ce que** le procédé présente en outre les étapes qui suivent consistant à :
(e) fournir une trempe ou un moût en tant qu'un deuxième milieu nutritif ; et
(f) mélanger le milieu obtenu à l'étape (b) et le deuxième milieu nutritif.

5. Procédé selon l'une quelconque des revendications 1 à 4, **caractérisé en ce que** le procédé présente en outre les étapes consistant à :
(i) de préférence affiner le milieu obtenu à l'étape (b) ou (f) ;
(k) cuire ou maintenir au chaud et de préférence houblonner le milieu obtenu à l'étape (f) ou (i) ;
(1) supprimer au moins en partie le dépôt issu du milieu obtenu à l'étape (k) ; et
(m) de préférence régler la température du milieu obtenu à l'étape (1) sur une température de fermentation.

6. Procédé selon l'une quelconque des revendications 1 à 5, **caractérisé en ce que** le procédé présente en outre l'étape consistant à :
(p) transformer le milieu obtenu lors d'une des étapes (b), (f), (k), (1) ou (m) en une boisson, de préférence traiter ledit milieu avec une levure du genre *Saccharomyces.*

7. Procédé selon l'une quelconque des revendications 1 à 6, **caractérisé en ce que** le procédé présente en outre l'étape consistant à :
(q) transformer le milieu obtenu lors d'une des étapes (b), (f), (k), (1) ou (m) ou la boisson obtenue lors de l'étape (p) en un milieu nutritif non fluide ;
dans lequel le milieu obtenu lors d'une des étapes (b), (f), (k), (1), ou (m) ou la boisson obtenue lors de l'étape (p) est mélangé(e) à un précurseur du milieu nutritif non fluide.

8. Procédé selon l'une quelconque des revendications 1 à 7, **caractérisé en ce que** le procédé présente en outre l'étape consistant à :
(t) régler une proportion massique en eau dans le milieu, qui a été obtenue lors d'une des étapes (b), (f), (k), (1) ou (m), dans la boisson obtenue à l'étape (p) ou du milieu nutritif non fluide obtenu lors de l'étape (q) sur au moins une valeur inférieure à 35 %, de préférence inférieure à 30 %, de préférence inférieure à 25 %, de préférence inférieure à 20 %, en particulier inférieure à 15 % ; et
de préférence sur une valeur supérieure à 0 %, en particulier supérieure à 5 %.

9. Milieu nutritif ou précurseur de ce dernier, fabriqué à l'aide d'un procédé selon l'une quelconque des revendications 1 à 8.

10. Utilisation d'un produit à base de levure dans un procédé servant à fabriquer un milieu nutritif ou un précurseur de ce dernier, de préférence dans un procédé selon l'une quelconque des revendications 1 à 8,
dans laquelle le procédé présente au moins les étapes consistant à :
(a) fournir une trempe ou un moût ou un dernier lavage en tant qu'un premier milieu nutritif ; et
(b) traiter le premier milieu nutritif avec des bactéries lactiques du type *Lactobacillus coryniformis,* du type *Lactobacillus backii* (DSMZ n° 18080), du type *Lactobacillus plantarum* ou du type *Lactobacillus fermentum* (DSMZ n° 20052) ou avec un mélange de bactéries lactiques d'au moins deux de ces types ;
dans laquelle le traitement selon l'étape (b) a lieu en présence d'un produit à base de levure ;
dans laquelle le produit à base de levure contient un extrait, un autolysat et/ou une forme séchée d'une levure.

11. Utilisation d'un procédé servant à fabriquer un agent nutritif ou un précurseur de ce dernier selon l'une quelconque des revendications 1 à 8 servant à augmenter la biodisponibilité et/ou la quantité produite et/ou la concentration massique en vitamine B₁₂ dans le milieu nutritif ou dans le précurseur de ce dernier ;
dans laquelle « vitamine B₁₂ » se limite à une variété sélectionnée parmi un groupe constitué de : méthylcobalamine, désoxyadénosylcobalamine, hydroxocobalamine et sulfitocobalamine.

12. Utilisation selon la revendication 10 ou 11, **caractérisée en ce que** le produit à base de levure est présent en une concentration massique située dans la plage ≥ 0,2 et ≤ 20 g/l par rapport au premier milieu nutritif.

13. Utilisation selon l'une quelconque des revendications 10 à 12, **caractérisée en ce que** le premier milieu nutritif est sans ou sensiblement sans houblon ; et/ou
le produit à base de levure est sans ou sensiblement sans houblon ;
dans laquelle « sans houblon » signifie, eu égard au premier milieu nutritif, l'absence totale de houblon dans le premier milieu nutritif ;
dans laquelle « sans houblon » signifie, eu égard au produit à base de levure, l'absence totale de houblon dans le produit à base de levure ;
dans laquelle « sensiblement sans houblon » signifie, eu égard au premier milieu nutritif, une teneur en houblon dans le premier milieu nutritif de maximum 15 % par rapport à la teneur en houblon, que présente un moût de fermentation utilisé habituellement dans les brasseries en vue d'une fermentation avec une levure à basse fermentation ou à haute fermentation présentant des unités d'amertume (méthode d'analyse de l'EBC) situées dans la plage allant de 15 à 38 ; et
dans laquelle « sensiblement sans houblon » signifie, eu égard au produit à base de levure, une teneur en houblon dans le produit à base de levure de maximum 20 % par rapport à la teneur en houblon, qu'une levure de service à basse fermentation ou à haute fermentation présente dans une brasserie, laquelle a été récoltée au cours d'une fermentation d'un moût habituellement utilisé dans les brasseries présentant des unités d'amertume (méthode d'analyse de l'EBC) situées dans la plage allant de 15 à 38.
